# EUROPÄISCHE PATENTSCHRIFT

(11) **EP 4 069 441 B1**
(45) Veröffentlichungstag und Bekanntmachung des Hinweises auf die Patenterteilung: **31.01.2024**
(21) Anmeldenummer: 20820381.0
(22) Anmeldetag: 04.12.2020
(51) Int. Cl.: A61L 2/18, B08B 3/02, B08B 13/00

(54) **REINIGUNG VON REINIGUNGSGUT MIT MINDESTENS EINEM HOHLRAUM IN EINER TRANSPORTSPÜLMASCHINE**
CLEANING ITEMS TO BE CLEANED, WHICH HAVE AT LEAST ONE CAVITY, IN A CONVEYOR WASHER
NETTOYAGE D'ARTICLES À NETTOYER, QUI PRÉSENTENT AU MOINS UNE CAVITÉ, DANS UNE LAVEUSE À CONVOYEUR

(30) Priorität: 06.12.2019 DE 102019219094
(43) Veröffentlichungstag der Anmeldung: 12.10.2022
(73) Patentinhaber: MEIKO Maschinenbau GmbH & Co. KG, 77652 Offenburg (DE)
(72) Erfinder: ECKER, Engelbert, 77654 Offenburg (DE); GAUS, Bruno, 77654 Offenburg (DE); HILS, Wendelin, 77836 Rheinmünster (DE); NÄGER, Thomas, 77652 Offenburg (DE); SCHERER, Marc, 77716 Hofstetten (DE); SIMUNDIC, Marijan, 77797 Ohlsbach (DE)
(74) Vertreter: Altmann Stößel Dick Patentanwälte PartG mbB
(86) Internationale Anmeldenummer: PCT/EP2020/084601
(87) Internationale Veröffentlichungsnummer: WO 2021/110897

(56) Entgegenhaltungen:
- EP-A1- 3 563 940
- DE-A1-102014 209 765
- GB-A- 1 380 740

## Beschreibung

### Technisches Gebiet

Die Erfindung betrifft einen Reinigungsgut-Träger, ein Reinigungssystem und ein Verfahren zur Reinigung von Reinigungsgut mit mindestens einem Hohlraum in einer Transportspülmaschine. Weiterhin betrifft die Erfindung eine Verwendung des erfindungsgemäßen Reinigungsgut-Trägers sowie des Reinigungssystems zur Reinigung von persönlicher Schutzausrüstung. Derartige Vorrichtungen und Verfahren können allgemein insbesondere eingesetzt werden, um Gegenstände zu reinigen, welche einen oder mehrere Hohlräume wie beispielsweise einen oder mehrere Kanäle aufweisen. Besonders hervorzuheben sind hier Schutzhelme, Atemgeräte oder andere Bestandteile persönlicher Schutzausrüstung, insbesondere Bestandteile persönlicher Schutzausrüstung, welche mindestens einen Kanal aufweisen, beispielsweise einen Kanal für eine Zufuhr oder Abfuhr von Atemluft. Insbesondere kann es sich hierbei um persönliche Schutzausrüstung für Produktionspersonal in toxischer und/oder staubbelasteter Umgebung handeln. Alternativ oder zusätzlich kann es sich auch um persönliche Schutzausrüstung wie beispielsweise Atemgeräte für Rettungskräfte wie Feuerwehr, technische Hilfsorganisationen oder Rettungssanitäter, Atemgeräte für Taucher oder allgemein Personen in lebensfeindlichen oder kritischen Arbeitsumgebungen sowie für Streitkräfte und Sicherheitskräfte wie beispielsweise Polizisten handeln. Auch für Atemgeräte im medizinischen Bereich, beispielsweise Atemmasken für Sauerstoffversorgung und Operationen, lassen sich die vorgeschlagenen Vorrichtungen und Verfahren einsetzen. Auch andere Einsatzgebiete sind denkbar, beispielsweise ein Einsatz bei der Reinigung von anderem Reinigungsgut mit mindestens einem Hohlraum, beispielsweise Flaschen, Karaffen, Gläsern oder ähnlichem Reinigungsgut.

### Technischer Hintergrund

Aus zahlreichen Anwendungen sind Gegenstände bekannt, welche mindestens einen Hohlraum aufweisen. Als Beispiel können hierbei Gegenstände genannt werden, welche einen oder mehrere Kanäle aufweisen, beispielsweise für eine Zufuhr und/oder Abfuhr von Luft. Auch andere Gegenstände können jedoch auf diese Weise grundsätzlich gereinigt werden. Die Erfindung wird im Folgenden, ohne Beschränkung weiterer möglicher Anwendungen, insbesondere beschrieben im Hinblick auf das in dieser Hinsicht besonders wichtige Gebiet der Reinigung von Gegenständen persönlicher Schutzausrüstung, insbesondere von Gegenständen mit mindestens einem Kanal für die Zufuhr und/oder Abfuhr von Atemluft.

So ist aus dem Stand der Technik eine Vielzahl von Atemgeräten für unterschiedliche Einsatzzwecke bekannt. Lediglich exemplarisch sind hier Atemgeräte wie beispielsweise Atemschutzmasken oder Lungenautomaten zu nennen, welche in der Regel Bestandteil der persönlichen Schutzausrüstung beispielsweise von Rettungskräften, Streitkräften oder Sicherheitskräften sind. Ein weiteres wichtiges Gebiet ist der Arbeitsschutz. Auch hier werden beispielsweise Helme oder Atemschutzmasken eingesetzt, welche eine Luftführung aufweisen, um Personal beispielsweise vor toxischen Stäuben oder Dämpfen zu schützen.

Diese Gegenstände der persönlichen Schutzausrüstung, einschließlich der Luftführung, müssen in der Regel nach jedem Einsatz gereinigt, hygienisiert, getrocknet, geprüft und gegebenenfalls instandgesetzt werden. Mit der Reinigung sollen alle Verschmutzungen infolge eines Gebrauchs oder einer Lagerung entfernt werden, so dass die Atemgeräte makroskopisch sauber und hygienisch einwandfrei bereitgestellt werden können, beispielsweise für die nächsten Schritte einer Aufbereitung.

Zahlreiche Reinigungsvorrichtungen sind bekannt, mittels derer Atemgeräte und Zubehör gereinigt werden können. Exemplarisch kann auf EP 0 935 687 B1, auf EP 1 088 928 A1, auf DE 200 03 743 U1 und auf DE 298 22 172 U1, auf DE 10 2005 033 618 B3, auf DE 200 03 744 U1, auf DE 10 2007 009 936 A1, auf DE 10 2007 012 768 B4, auf DE 100 20 835 A1, auf US 3,881,503 A, auf DE 11 74 169 B oder auf WO 2011/144518 A2 verwiesen werden. Auch aus anderen Bereichen der Technik sind grundsätzlich Reinigungsvorrichtungen bekannt, mittels derer kritische Gegenstände gereinigt werden können, wie beispielsweise chirurgische Instrumente. Diesbezüglich kann beispielsweise auf DE 27 12 020 A1 verwiesen werden, in welcher eine Dekontaminationsanlage für chirurgische Instrumente beschrieben wird.

GB 1 380 740 A beschreibt eine Maschine, die einen Korbkörper umfasst, der mit Stützmitteln zum Stützen von zu waschenden Gegenständen ausgestattet ist, wobei die Stützmittel Kanäle umfassen, die an der Oberseite und der Unterseite offen sind und sich im Gebrauch innerhalb des Korbkörpers nach oben erstrecken, wobei sich die Kanäle an ihren unteren Enden im Wesentlichen trichterförmig nach außen erweitern, wenn sie sich nach unten erstrecken.

Trotz der Vorteile, welche mit den oben beschriebenen Vorrichtungen und Verfahren erzielt wurden und werden, verbleiben zahlreiche technische Herausforderungen. Eine technische Herausforderung besteht insbesondere bei Gegenständen, beispielsweise persönlicher Schutzausrüstung, mit mindestens einem Hohlraum, insbesondere mindestens einem Kanal zur Luftführung, in einer Reinigung der Innenwände des Hohlraums. Zwar ist es grundsätzlich möglich, den Hohlraum separat mit Reinigungsfluid auszuspritzen, wie beispielsweise mit einer separaten Düse oder insbesondere auch manuell. Schwierigkeiten ergeben sich jedoch in der Praxis insbesondere dann, wenn ein hoher Durchsatz im Hinblick auf das Reinigungsgut erforderlich ist. So fallen beispielsweise bei Einsätzen von Rettungskräften oder auch im industriellen Umfeld und im Bereich des Arbeitsschutzes Gegenstände der persönlichen Schutzausrüstung in hoher Stückzahl an. So existieren Betriebe, in denen mehrere hundert bis hin zu tausenden verschmutzter Atemschutzmasken oder anderer Teile persönlicher Schutzausrüstung pro Arbeitsschicht, beispielsweise pro 10 Stunden, anfallen. Der Personalaufwand für die manuelle Reinigung derartiger Stückzahlen wäre erheblich, und die Qualität manueller Reinigung ist zudem oftmals starken Schwankungen unterworfen.

Bekannte Maskenspülmaschinen, beispielsweise gemäß den oben beschriebenen Ausgestaltungen, sind jedoch in der Regel nicht in der Lage, große Stückzahlen an Reinigungsgut zu reinigen. Transportspülmaschinen hingegen sind in der Regel nicht in der Lage, in ausreichender Weise und zuverlässig Hohlräume zu reinigen, wie sie insbesondere im Bereich der Kanäle der Luftführung auftreten, da nur ein geringer Anteil des Reinigungsfluids und zudem auch dieser geringe Anteil nur unzuverlässig in die Hohlräume, beispielsweise die Kanäle, eintritt, wenn das Reinigungsgut die stationären Düsen passiert. So müssten allgemein mehrere Einkammer-Maskenspülmaschinen parallel betrieben werden, um den Durchsatz zu ermöglichen, wodurch der Verbrauch an Ressourcen und die Anschaffungskosten jedoch linear ansteigen würden. Zudem müsste, je nach Art und Menge der Verschmutzung der persönlichen Schutzausrüstung, das Abwasser aus den Maskenspülmaschinen separat als Sondermüll entsorgt werden, wodurch zusätzliche Kosten und Umweltbelastungen anfallen könnten. Auch Lösungen, welche auf Transportspülmaschinen basieren, weisen in der Praxis erhebliche Probleme auf. So ist beispielsweise der in DE 27 12 020 A1 beschriebene Anschluss eines Düsenkorbs an eine Zuführungsleitung der Transportspülmaschine mit einem hohen manuellen Aufwand der Installation verbunden, da jeweils am Einlauf und am Auslauf der Transportspülmaschine Installationsarbeiten erforderlich sind. Auch diese beschränken den Durchsatz erheblich und erfordern zudem einen hohen apparativen Aufwand.

### Aufgabe der Erfindung

Es wäre daher wünschenswert, Vorrichtungen und Verfahren zur Reinigung von Reinigungsgut mit mindestens einem Hohlraum bereitzustellen, welche die Nachteile bekannter Vorrichtungen und Verfahren der genannten Art zumindest weitgehend vermeiden. Insbesondere sollen die Vorrichtungen und Verfahren für die Reinigung von persönlicher Schutzausrüstung mit mindestens einem Hohlraum, beispielsweise mindestens einem Luftführungskanal, einsetzbar sein. Dabei soll mit möglichst geringem Personalaufwand und möglichst geringem technischen Aufwand das Reinigungsgut zuverlässig und sicher auch innerhalb des Hohlraums gereinigt werden, bei gleichzeitig hohem Durchsatz.

### Allgemeine Beschreibung der Erfindung

Diese Aufgabe wird adressiert durch einen Reinigungsgut-Träger, ein Reinigungssystem und ein Verfahren zur Reinigung von Reinigungsgut mit mindestens einem Hohlraum in einer Transportspülmaschine, sowie weiterhin durch eine Verwendung. Weiterhin betrifft die Erfindung eine Verwendung des erfindungsgemäßen Reinigungsgut-Trägers sowie des Reinigungssystems zur Reinigung von persönlicher Schutzausrüstung, mit den Merkmalen der unabhängigen Patentansprüche. Vorteilhafte Weiterbildungen, welche einzeln oder in beliebiger Kombination realisierbar sind, sind in den abhängigen Ansprüchen dargestellt.

Im Folgenden werden die Begriffe "haben", "aufweisen", "umfassen" oder "einschließen" oder beliebige grammatikalische Abweichungen davon in nicht-ausschließlicher Weise verwendet. Dementsprechend können sich diese Begriffe sowohl auf Situationen beziehen, in welchen, neben den durch diese Begriffe eingeführten Merkmalen, keine weiteren Merkmale vorhanden sind, oder auf Situationen, in welchen ein oder mehrere weitere Merkmale vorhanden sind. Beispielsweise kann sich der Ausdruck "A hat B", "A weist B auf', "A umfasst B" oder "A schließt B ein" sowohl auf die Situation beziehen, in welcher, abgesehen von B, kein weiteres Element in A vorhanden ist (d.h. auf eine Situation, in welcher A ausschließlich aus B besteht), als auch auf die Situation, in welcher, zusätzlich zu B, ein oder mehrere weitere Elemente in A vorhanden sind, beispielsweise Element C, Elemente C und D oder sogar weitere Elemente.

Weiterhin wird darauf hingewiesen, dass die Begriffe "mindestens ein" und "ein oder mehrere" sowie grammatikalische Abwandlungen dieser Begriffe, wenn diese in Zusammenhang mit einem oder mehreren Elementen oder Merkmalen verwendet werden und ausdrücken sollen, dass das Element oder Merkmal einfach oder mehrfach vorgesehen sein kann, in der Regel lediglich einmalig verwendet werden, beispielsweise bei der erstmaligen Einführung des Merkmals oder Elementes. Bei einer nachfolgenden erneuten Erwähnung des Merkmals oder Elementes wird der entsprechende Begriff "mindestens ein" oder "ein oder mehrere" in der Regel nicht mehr verwendet, ohne Einschränkung der Möglichkeit, dass das Merkmal oder Element einfach oder mehrfach vorgesehen sein kann.

Weiterhin werden im Folgenden die Begriffe "vorzugsweise", "insbesondere", "beispielsweise" oder ähnliche Begriffe in Verbindung mit optionalen Merkmalen verwendet, ohne dass alternative Ausführungsformen hierdurch beschränkt werden. So sind Merkmale, welche durch diese Begriffe eingeleitet werden, optionale Merkmale, und es ist nicht beabsichtigt, durch diese Merkmale den Schutzumfang der Ansprüche und insbesondere der unabhängigen Ansprüche einzuschränken. So kann die Erfindung, wie der Fachmann erkennen wird, auch unter Verwendung anderer Ausgestaltungen durchgeführt werden. In ähnlicher Weise werden Merkmale, welche durch "in einer Ausführungsform der Erfindung" oder durch "in einem Ausführungsbeispiel der Erfindung" eingeleitet werden, als optionale Merkmale verstanden, ohne dass hierdurch alternative Ausgestaltungen oder der Schutzumfang der unabhängigen Ansprüche eingeschränkt werden soll. Weiterhin sollen durch diese einleitenden Ausdrücke sämtliche Möglichkeiten, die hierdurch eingeleiteten Merkmale mit anderen Merkmalen zu kombinieren, seien es optionale oder nicht-optionale Merkmale, unangetastet bleiben.

In einem ersten Aspekt der vorliegenden Erfindung wird ein Reinigungsgut-Träger zur Reinigung von Reinigungsgut in einer Transportspülmaschine vorgeschlagen, wobei das Reinigungsgut mindestens einen Hohlraum aufweist. Insbesondere wird der Reinigungsgut-Träger zur Reinigung von Hohlräumen in persönlicher Schutzausrüstung eingesetzt. Auch andere Einsatzgebiete sind jedoch grundsätzlich möglich.

Der Begriff "Reinigungsgut-Träger", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine grundsätzlich beliebige Vorrichtung beziehen, welche eingerichtet ist, um während einer Reinigung Reinigungsgut zu halten, insbesondere in einer vorgegebenen räumlichen Positionierung und/oder Orientierung. Der Reinigungsgut-Träger kann dabei selbst gereinigt werden. Der Reinigungsgut-Träger kann beispielsweise ganz oder teilweise aus mindestens einem Kunststoffmaterial hergestellt sein. Insbesondere kann der Reinigungsgut-Träger durch mindestens ein additives Fertigungsverfahren hergestellt sein, beispielsweise durch mindestens ein 3D-Druckverfahren und/oder durch Kunststoff-Lasersintern. Alternativ oder zusätzlich sind jedoch auch andere Materialien und/oder Herstellungsverfahren möglich, beispielsweise metallische Materialien.

Der Begriff "Reinigung", wie er hier verwendet wird, ist ebenfalls ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf einen Vorgang beziehen, bei welchem Reinigungsgut von anhaftenden makroskopischen und/oder mikroskopischen Verunreinigungen befreit wird und/oder bei welchem derartige Verunreinigungen zumindest teilweise beseitigt werden. Zusätzlich kann optional eine Desinfektionswirkung ausgeübt werden.

Der Begriff "Reinigungsgut", wie er hier verwendet wird, ist ebenfalls ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf beliebige Gegenstände beziehen, welche der Reinigung unterworfen werden sollen. Insbesondere kann es sich dabei, wie ausgeführt, um Gegenstände handeln, welche mindestens einen Hohlraum aufweisen, insbesondere Reinigungsgut mit mindestens einem Kanal, insbesondere mindestens einem Kanal mit mindestens einer Eintrittsöffnung und beispielsweise auch mindestens einer Austrittsöffnung. Insbesondere umfasst das Reinigungsgut, wie unten noch näher ausgeführt wird, persönliche Schutzausrüstung. Insbesondere kann das Reinigungsgut somit mindestens eine Atemschutzmaske umfassen, auch als Atemmaske bezeichnet. Der Begriff "Atemschutzmaske", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine Vorrichtung beziehen, welche dazu eingerichtet ist, das Gesicht eines Benutzers ganz oder teilweise zu bedecken, insbesondere eine Mundpartie und/oder Nasenpartie. Die Bedeckung kann insbesondere dem Schutz der Atemwege des Benutzers vor Schadstoffen dienen und/oder der Einleitung von Atemgasen in die Atemwege und/oder der Ausleitung von Atemgasen aus den Atemwegen. Die Atemschutzmaske kann darüber hinaus weitere Partien des Gesichts und/oder des Kopfes des Benutzers bedecken, beispielsweise die Augen oder eine obere Partie des Kopfes. Dementsprechend kann die Atemschutzmaske insbesondere auch mindestens ein transparentes Sichtfenster aufweisen. Die Atemschutzmaske kann insbesondere mindestens einen Kanal für eine Luftführung von Atemluft, insbesondere Zuluft und/oder Abluft, aufweisen. Die Atemschutzmaske kann ganz oder teilweise starr oder auch ganz oder teilweise flexibel ausgebildet sein. So kann die Atemschutzmaske auch beispielsweise ganz oder teilweise als Helm ausgestaltet sein. Alternativ oder zusätzlich kann die Atemschutzmaske auch ganz oder teilweise flexibel sein, wie beispielsweise dies bei Vollschutzmasken für Rettungskräfte der Fall ist. Weiterhin kann die Atemschutzmaske mindestens eine Fixierung zur Befestigung am Kopf des Benutzers aufweisen, beispielsweise mindestens einen Riemen, mindestens ein Fixierband oder mindestens eine Befestigung, die aus mehreren Bändern besteht. Die Atemschutzmaske kann beispielsweise mindestens eine Öffnung aufweisen und kann beispielsweise eingerichtet sein, um eine Luftzufuhr oder Atemgaszufuhr zu ermöglichen. Beispielsweise kann die Atemschutzmaske mindestens ein Gewinde zum Anschluss einer Atemgaszufuhr aufweisen. Weiterhin kann die Atemschutzmaske beispielsweise mindestens ein Ausatemventil aufweisen. Darüber hinaus kann die Atemschutzmaske weitere Komponenten umfassen.

Der Begriff "Spülmaschine", auch allgemein als "Reinigungsvorrichtung" bezeichnet, wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine Vorrichtung beziehen, welche eingerichtet ist, um Reinigungsgut von anhaftenden makroskopischen oder auch mikroskopischen Verunreinigungen zu befreien oder derartige Verunreinigungen zumindest teilweise zu beseitigen. Zusätzlich kann optional eine Desinfektionswirkung ausgeübt werden.

Dementsprechend ist der Begriff "Transportspülmaschine", wie er hier verwendet wird, ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine Spülmaschine im Sinne der obigen Definition beziehen, welche mindestens eine Transportvorrichtung aufweist. Insbesondere kann die Transportspülmaschine, wie unten noch näher ausgeführt wird, mindestens eine Reinigungskammer aufweisen, beispielsweise mindestens einen Reinigungstunnel, in welcher das Reinigungsgut mit mindestens einem Reinigungsfluid beaufschlagt wird, insbesondere mittels mindestens einer Beaufschlagungsvorrichtung, wie unten ebenfalls noch näher erläutert wird. Die Transportvorrichtung kann eingerichtet sein, um das Reinigungsgut durch die Reinigungskammer hindurch zu transportieren.

Der Reinigungsgut-Träger umfasst mindestens ein Basiselement. Der Begriff "Basiselement", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf ein Element beziehen, welches eingerichtet ist, um ein oder mehrere weitere Elemente zu tragen, zu positionieren oder in sonstiger Weise mechanisch zu unterstützen. Das Basiselement kann insbesondere ganz oder teilweise als ebenes und/oder plattenförmiges Element ausgestaltet sein. Das Basiselement kann beispielsweise ganz oder teilweise als ebenes, gitterförmiges Element ausgestaltet sein. Beispiele werden unten noch näher erläutert. Das Basiselement kann beispielsweise ganz oder teilweise aus Kunststoff hergestellt sein. Auch eine vollständige oder teilweise Herstellung aus metallischen Materialien ist grundsätzlich möglich.

Der Reinigungsgut-Träger umfasst weiterhin mindestens ein Düsenrohr mit mindestens einer Eintrittsöffnung und mindestens einer Austrittsdüse. Dieses mindestens eine Düsenrohr ist mit dem Basiselement verbunden. Beispielsweise kann das Düsenrohr das Basiselement durchdringen. Alternativ oder zusätzlich kann das Düsenrohr jedoch auch ganz oder teilweise in das Basiselement integriert sein und/oder von dem Basiselement gehalten werden. Beispielsweise kann das Düsenrohr das Basiselement von einer Seite, auf welcher die Eintrittsöffnung angeordnet ist, bis hin zu der Seite, auf welcher die Austrittsdüse angeordnet ist, durchdringen. Beispielsweise kann die Eintrittsöffnung auf einer Unterseite des Basiselements angeordnet sein, und die Austrittsdüse auf einer, der Unterseite des Basiselements gegenüberliegenden, Oberseite. Alternativ kann die Eintrittsöffnung jedoch auch beispielsweise auf mindestens einer Längsseite angeordnet sein oder auch auf der Oberseite selbst. Ohne Beschränkung weiterer möglicher Ausgestaltungen wird im Folgenden davon ausgegangen, dass die Eintrittsöffnung auf der Unterseite des Basiselements angeordnet ist und die Austrittsdüse auf der, der Unterseite des Basiselements gegenüberliegenden, Oberseite und dass das Düsenrohr das Basiselement durchdringt.

Das Düsenrohr weist, wie ausgeführt, mindestens eine Eintrittsöffnung auf. Diese Eintrittsöffnung weist mindestens einen Fangtrichter auf. Das Düsenrohr verjüngt sich von der Eintrittsöffnung hin zu der Austrittsdüse zumindest abschnittsweise.

Der Begriff "Düse", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine Vorrichtung beziehen, welche mindestens eine Öffnung in einem Fluid-führenden Element aufweist, beispielsweise einem Rohr oder Düsenarm, aus welcher Fluid aus dem Fluid-führenden Element austreten kann, insbesondere als Strahl. Die Öffnung kann beispielsweise als runde Bohrung ausgestaltet sein, oder auch als Bohrung mit einem polygonalen, ovalen oder linienförmigen Querschnitt. Auch andere Querschnitte sind möglich. Eine Düse kann dabei eine einzelne Öffnung aufweisen oder auch eine Mehrzahl von Öffnungen, So kann sich an der Düse beispielsweise ein einzelner Strahl bilden, oder auch eine Mehrzahl paralleler oder auch nichtparalleler Strahlen, beispielsweise ein Strahlenbündel.

Der Begriff "Austrittsdüse", wie er hier verwendet wird, ist dementsprechend ebenfalls ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine Düse im Sinne der vorhergehenden Definition beziehen, welche in dem Düsenrohr vorgesehen ist und durch welche Reinigungsfluid, welches durch den Fangtrichter in das Düsenrohr eintritt, wieder austreten kann.

Der Begriff "Düsenrohr", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf einen Hohlkörper beziehen, beispielsweise ein Rohr, einen Kanal oder eine Kanalstruktur, welche mindestens eine Düsenöffnung aufweist, aus welcher fluides Medium austreten kann, beispielsweise als Strahl. Das Düsenrohr kann beispielsweise ganz oder teilweise aus mindestens einem Kunststoffmaterial hergestellt sein. Alternativ oder zusätzlich sind jedoch auch beispielsweise metallische Materialien möglich. Das Düsenrohr kann, wie oben ausgeführt, separat von dem Basiselement ausgestaltet sein oder kann auch ganz oder teilweise in dasselbe integriert sein oder von dem Basiselement gehalten werden.

Das Basiselement weist, wie oben ausgeführt, insbesondere mindestens eine Unterseite und mindestens eine Oberseite auf. Die Oberseite kann insbesondere die Seite sein, auf der das Reinigungsgut aufgenommen wird, wohingegen die Unterseite beispielsweise der Transportvorrichtung zuweisen kann. Die Oberseite und die Unterseite können beispielsweise zueinander parallele Seiten sein. Beispielsweise kann das Basiselement als plattenförmiges Element ausgestaltet sein, mit einer ebenen Unterseite und einer ebenen Oberseite.

Der Begriff "Fangtrichter", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine trichterförmige Vorrichtung am Eingang eines Hohlraums oder Rohres beziehen, welche eingerichtet ist, fluide Medien in den Hohlraum und/oder das Rohr zu leiten. Der Fangtrichter kann sich dabei gleichmäßig oder ungleichmäßig in seinem Querschnitt oder Äquivalentdurchmesser verjüngen. Das Düsenrohr verjüngt sich, wie oben ausgeführt, von der Eintrittsöffnung zu der Austrittsdüse hin zumindest abschnittsweise. Der Fangtrichter kann dabei ein Teil dieses sich verjüngenden Düsenrohres sein. Die Verjüngung des Düsenrohres kann gleichmäßig erfolgen, beispielsweise indem ein Durchmesser oder Äquivalentdurchmesser eine monoton oder streng monoton fallende Funktion einer Wegstrecke von der Eintrittsöffnung zu der Austrittsöffnung bildet. Alternativ kann sich das Düsenrohr jedoch auch lediglich in einem oder mehreren Abschnitten verjüngen. Weiterhin ist an der Austrittsdüse auch wiederum eine Aufweitung möglich, beispielsweise zur Strahlformung.

Der Reinigungsgut-Träger kann insbesondere ausgestaltet sein in einer oder mehreren der folgenden Weisen: einem Kettenglied einer Transportkette einer Transportvorrichtung einer Transportspülmaschine; einem Reinigungskorb, wobei das Basiselement Teil eines Korbbodens des Reinigungskorbs bildet. So kann der Reinigungsgut-Träger auch ganz oder teilweise in eine Transportkette der Transportvorrichtung integriert sein. Alternativ kann der Reinigungsgut-Träger auch auf die Transportvorrichtung, beispielsweise ein Transportband oder eine Transportkette, aufgesetzt werden, beispielsweise als Reinigungskorb.

Wie oben ausgeführt, kann das Düsenrohr an der Austrittsdüse insbesondere strahlformende Eigenschaften aufweisen. Insbesondere kann der Reinigungsgut-Träger mindestens ein strömungsformendes Element aufweisen, welches eingerichtet ist, um eine aus der Austrittsdüse austretende Strömung des Reinigungsfluids zu formen. Diese Strahlformung kann eine Umlenkung umfassen, eine Bündelung, eine Aufweitung, eine Verleihung eines Drehimpulses, eine Aufteilung oder andere Arten der Strahlformung oder auch Kombinationen der genannten Arten. Das strömungsformende Element kann insbesondere ausgewählt sein aus der Gruppe bestehend aus: einem Trichter; einer Düse; einer Leitschaufel; einer Rohrleitung; einem Drall-induzierenden Element.

Wie oben ausgeführt, kann der Reinigungsgut-Träger insbesondere ganz oder teilweise in eine Gliederkette integrierbar sein. Insbesondere kann das Basiselement ganz oder teilweise in diese Gliederkette integrierbar sein, wobei diese Gliederkette beispielsweise Bestandteil der Transportvorrichtung der Transportspülmaschine sein kann. Diese Integration in die Gliederkette kann vorübergehend und lösbar oder auch permanent sein. Weiterhin können insbesondere auch lediglich Teile des Reinigungsgut-Trägers in die Gliederkette integriert sein, beispielsweise lediglich das Basiselement, wobei ein oder mehrere weitere, mit dem Basiselement verbindbare Teile wieder von dem Basiselement gelöst werden können, beispielsweise am Auslauf der Transportspülmaschine. Insbesondere kann das Basiselement zumindest teilweise als Kettenglied einer Gliederkette ausgebildet sein, insbesondere mit Formelementen in denen sich mindestens zwei vorzugsweise vier Bohrungen befinden, durch die die Bandstangen des Transportbandes beziehungsweise der Transportkette hindurchgeführt werden können.

Wie oben ausgeführt, kann das Düsenrohr vollständig oder teilweise verjüngt ausgebildet sein. So ist es zum Beispiel auch möglich, dass das Düsenrohr von der Eintrittsöffnung hin zu der Austrittsdüse trichterförmig ausgestaltet ist.

Weiterhin kann insbesondere die Eintrittsöffnung einen Äquivalentdurchmesser aufweisen, welcher größer ist als ein Durchmesser oder Äquivalentdurchmesser der Austrittsdüse. Insbesondere kann die Eintrittsöffnung einen Äquivalentdurchmesser aufweisen, welcher um mindestens einen Faktor 1,3, insbesondere um mindestens einen Faktor 1,5, insbesondere um mindestens einen Faktor 2 größer ist als ein Äquivalentdurchmesser der Austrittsdüse.

Die Eintrittsöffnung kann rund ausgestaltet sein, wobei eine runde Gestalt nicht zwingend erforderlich ist. Insbesondere kann die Eintrittsöffnung auch, wie nachfolgend noch näher ausgeführt wird, länglich ausgebildet sein, insbesondere um einer Bewegung des Reinigungsgut-Trägers relativ zu mindestens einer ortsfest in der Transportspülmaschine angeordneten Wurfdüse Rechnung zu tragen. Insbesondere kann insgesamt die Eintrittsöffnung eine Gestalt aufweisen, ausgewählt aus der Gruppe bestehend aus: einer kreisförmigen Gestalt; einer ovalen Gestalt, insbesondere einer ovalen Gestalt mit einer Längserstreckung in einer Transportrichtung der Transportspülmaschine; einer polygonalen Gestalt.

Das Düsenrohr kann insbesondere auf der Unterseite des Basiselements bündig mit dem Basiselement abschließen. Auf diese Weise kann beispielsweise, indem das Düsenrohr nicht über die Unterseite hervorsteht, eine Kollision des Düsenrohres mit Bestandteilen der Transportspülmaschine vermieden werden. Auf der Oberseite kann das Düsenrohr jedoch über das Basiselement hinausragen. So kann das Düsenrohr dort insbesondere einen Stutzen bilden, über welchen Reinigungsfluid in den Hohlraum des Reinigungsguts eindringen kann. Eine Mündung dieses Stutzens kann beispielsweise die Austrittsdüse bilden, welche mit einer Öffnung des Hohlraums des Reinigungsguts zur Überdeckung gebracht werden kann, um das Reinigungsfluid direkt oder indirekt in die Öffnung einzuspritzen.

Wie oben ausgeführt, kann die Eintrittsöffnung des Düsenrohres insbesondere an der Unterseite des Basiselements angeordnet sein. Alternativ oder zusätzlich sind jedoch auch andere Anordnungen für die mindestens eine Eintrittsöffnung möglich. So kann beispielsweise auch mindestens eine Eintrittsöffnung an mindestens einer Seitenwand des Reinigungsgut-Trägers angeordnet sein, insbesondere an mindestens einer Längswand, welche parallel zu einer Transportrichtung der Transportspülmaschine angeordnet ist. Weiterhin ist grundsätzlich auch eine Anordnung auf der Oberseite des Reinigungsgut-Trägers möglich, beispielsweise auf der Oberseite des Basiselements. Insgesamt kann damit die mindestens eine Eintrittsöffnung insbesondere ausgewählt sein aus der Gruppe bestehend aus: einer auf der Unterseite des Basiselements angeordneten Eintrittsöffnung; einer an einer Längsseite des Basiselements angeordneten Eintrittsöffnung; einer auf der Oberseite des Basiselements angeordneten Eintrittsöffnung. Auch andere Ausgestaltungen sind jedoch grundsätzlich möglich.

Das Basiselement kann als solides, plattenförmiges Element ohne Öffnungen ausgestaltet sein. Zum Zweck des Ablaufs des Reinigungsfluids sowie auch zur weiteren Unterstützung des Reinigungsvorgangs kann es jedoch vorteilhaft sein, wenn der Reinigungsgut-Träger und insbesondere das Basiselement eine oder mehrere Öffnungen aufweisen. So können der Reinigungsgut-Träger und/oder das Basiselement insbesondere ganz oder teilweise als Gitter ausgestaltet sein. Insbesondere kann das Basiselement zumindest teilweise als Gitter ausgestaltet sein. Das Düsenrohr kann dann beispielsweise durch Gitterstreben mit einem Rahmen des Basiselements verbunden sein.

Das Düsenrohr kann sich insbesondere im Wesentlichen senkrecht zu dem Basiselement erstrecken, beispielsweise zu einer Ebene des Basiselements. Dabei kann eine Winkelstellung von exakt 90° auftreten, oder auch eine Abweichung von 90°, beispielsweise um nicht mehr als 20°. Das Düsenrohr kann als gerades Düsenrohr ausgestaltet sein, mit einer geraden Rohrachse. Alternativ oder zusätzlich kann das Düsenrohr jedoch auch in einem oder mehreren Abschnitten gekrümmt ausgebildet sein, beispielsweise mit einer gekrümmten Rohrachse.

Wie oben ausgeführt, kann das Basiselement insbesondere im Wesentlichen eben ausgestaltet sein. So kann das Basiselement beispielsweise eine plattenförmige Gestalt aufweisen. Beispielsweise kann das Basiselement eine Grundform eines Rechtecks aufweisen, mit einer konstanten Dicke. Diese Plattenform kann, wie oben ausgeführt, auch eine Gitterform sein.

Wie oben ausgeführt, kann das Basiselement als Träger für ein oder mehrere weitere Elemente des Reinigungsgut-Trägers fungieren. Diese Elemente können fest oder lösbar mit dem Basiselement verbunden sein. Insbesondere kann auf dem Basiselement mindestens ein Gestell aufgebracht sein. Dieses Gestell, welches beispielsweise aus einem Kunststoffmaterial und/oder einem metallischen Material hergestellt sein kann, kann mehreren Zwecken dienen. So kann dieses Gestell beispielsweise eine Halterung für das Reinigungsgut und/oder eine Fixierung für das Reinigungsgut oder Teile desselben bereitstellen. Weiterhin kann das Gestell auch, alternativ oder zusätzlich, einen Rahmen bilden. Das Basiselement kann beispielsweise eine ebene Auflagefläche bilden, und das Gestell kann abnehmbar mit dem Basiselement verbunden sein. Auf diese Weise können beispielsweise das Basiselement und das Gestell gemeinsam einen Reinigungskorb bilden. Das Gestell kann insbesondere abnehmbar mit dem Basiselement verbunden sein.

Das Gestell kann, wie oben ausgeführt, insbesondere mindestens eine Halterung für das Reinigungsgut bilden. So kann das Gestell beispielsweise mindestens eine Halterung für einen Helm und/oder eine Atemschutzmaske bilden. Dabei kann das Gestell zusätzliche Aufgaben übernehmen. So kann das Gestell beispielsweise weiterhin mindestens eine Halterung für ein Visier des Helms bilden oder aufweisen. Insbesondere kann diese Halterung eingerichtet sein, um das Visier in einer vorgegebenen Winkelstellung relativ zum übrigen Helm zu halten. In dem Gestell können ein oder mehrere Helme und/oder eine oder mehrere Atemschutzmasken aufgenommen sein.

Bei der Reinigung persönlicher Schutzausrüstung können Situationen auftreten, in denen nicht alle Bereiche des Reinigungsguts mit Reinigungsfluid beaufschlagt werden dürfen. So sind insbesondere komplexere Helme bekannt, bei denen Ventile oder auch elektronische Komponenten nicht mit Reinigungsfluid in Kontakt geraten dürfen. So kann der Reinigungsgut-Träger beispielsweise mindestens ein beweglich zu dem Basiselement angeordnetes Verschlusselement aufweisen, welches eingerichtet ist, um mindestens eine Öffnung des Reinigungsguts während der Reinigung und während der Beaufschlagung des Reinigungsguts mit Reinigungsfluid zu verschließen. Insbesondere kann diese mindestens eine Öffnung ausgewählt sein aus der Gruppe bestehend aus einer elektrischen Anschlussöffnung und einer Luftführungsöffnung. Das Verschlusselement kann beispielsweise aus einem verformbaren, insbesondere flexiblen Material hergestellt sein, beispielsweise Silikon oder Gummi. Das Verschlusselement kann insbesondere durch mindestens ein flexibles Verbindungselement mit dem Basiselement verbunden sein, beispielsweise eine Kordel und/oder einen flexiblen, langgestreckten Kunststoffsteg. Auch andere Ausgestaltungen sind möglich.

Der Reinigungsgut-Träger kann insbesondere mindestens einen Identifikator aufweisen. Der Begriff "Identifikator", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine Vorrichtung beziehen, welche mit einer anderen Vorrichtung oder einem anderen Element verbunden werden kann und welche es erlaubt, die andere Vorrichtung oder das andere Element zu identifizieren. Die Identifikation kann dabei insbesondere automatisch erfolgen. Insbesondere kann der mindestens eine Identifikator ausgewählt sein aus der Gruppe bestehend aus: einem metallischen Identifikator, einem induktiven Identifikator, einem RFID-Identifikator; einem grafischen Identifikator, insbesondere einem Barcode und/oder QR-Code. Auch andere Identifikatoren sind jedoch grundsätzlich einsetzbar.

In einem weiteren Aspekt der vorliegenden Erfindung wird ein Reinigungssystem vorgeschlagen, welches zur Reinigung von Reinigungsgut mit mindestens einem Hohlraum eingerichtet ist, insbesondere zur Reinigung von persönlicher Schutzausrüstung. Der Begriff "Reinigungssystem", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf ein System beziehen, welches zur Reinigung von Reinigungsgut eingerichtet ist. Dabei ist der Begriff "System", wie er hier verwendet wird, ebenfalls ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine aus mindestens zwei Komponenten zusammengesetzte Einheit beziehen, wobei die Komponenten eingerichtet sind, um funktionell zur Erreichung mindestens eines Zwecks des Systems zusammen zu wirken. Die Komponenten können dabei miteinander mechanisch und/oder elektrisch verbunden sein, insbesondere reversibel.

Das Reinigungssystem umfasst folgende Komponenten:
I. mindestens eine Transportspülmaschine, umfassend:
   - mindestens eine Reinigungskammer,
   - mindestens eine Beaufschlagungsvorrichtung zur Beaufschlagung des Reinigungsguts in der Reinigungskammer mit mindestens einem Reinigungsfluid,
   - mindestens eine Transportvorrichtung zum Transport des Reinigungsguts von einem Einlauf der Transportspülmaschine durch die Reinigungskammer hindurch zu einem Auslauf der Transportspülmaschine; und
II. mindestens einen Reinigungsgut-Träger gemäß der vorliegenden Erfindung, beispielsweise gemäß einer oder mehreren der oben beschriebenen Ausgestaltungen und/oder gemäß einer oder mehreren der nachfolgend noch näher beschriebenen Ausführungsformen.

Dabei ist das Reinigungsgut derart auf dem Reinigungsgut-Träger aufnehmbar, dass der Hohlraum des Reinigungsguts mit der Austrittsdüse des Reinigungsgut-Trägers in Verbindung steht. Der Reinigungsgut-Träger ist mittels der Transportvorrichtung durch die Reinigungskammer transportierbar. Die Beaufschlagungsvorrichtung weist mindestens eine stationäre Wurfdüse auf. Diese mindestens eine Wurfdüse ist in mindestens einer Stellung des Reinigungsgut-Trägers derart relativ zu dem Reinigungsgut-Träger positioniert, dass aus der Wurfdüse austretendes Reinigungsfluid in den Fangtrichter des Düsenrohrs eintritt.

Der Begriff "Reinigungskammer", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine vollständig oder teilweise geschlossene Kammer beziehen, innerhalb derer der Reinigungsvorgang vollständig oder teilweise durchgeführt werden kann. Die Reinigungskammer kann insbesondere mindestens ein Gehäuse aufweisen, welches die Reinigungskammer ganz oder teilweise umschließt. Dabei kann eine einzige Reinigungskammer vorgesehen sein, oder es können grundsätzlich auch mehrere Reinigungskammern, beispielsweise sequenziell, vorgesehen sein. Insbesondere kann die Reinigungskammer beispielsweise ganz oder teilweise als Tunnel ausgestaltet sein, beispielsweise als Tunnel mit einer Einlauföffnung und einer Auslauföffnung. Dementsprechend können sich die Begriffe "Einlauf" und "Auslauf' auf Bereiche der Transportspülmaschine und/oder der Transportvorrichtung der Transportspülmaschine beziehen, in denen das Reinigungsgut in die Reinigungskammer eintritt bzw. aus der Reinigungskammer austritt. In diesen Bereichen kann beispielsweise die Transportvorrichtung über die Reinigungskammer hinausragen und frei zugänglich sein.

Die Transportspülmaschine ist eingerichtet, um das Reinigungsgut in der Reinigungskammer mit mindestens einem Reinigungsfluid zu beaufschlagen. Zu diesem Zweck kann die Transportspülmaschine insbesondere die mindestens eine Beaufschlagungsvorrichtung aufweisen, insbesondere innerhalb der Reinigungskammer. Der Begriff "Beaufschlagungsvorrichtung", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine grundsätzlich beliebige Vorrichtung beziehen, mittels derer das Reinigungsgut innerhalb der Reinigungskammer mit der Reinigungsflüssigkeit beaufschlagt werden kann. Die Beaufschlagungsvorrichtung kann insbesondere mindestens ein Düsensystem umfassen. Weiterhin kann die Beaufschlagungsvorrichtung mindestens eine Pumpe umfassen, sowie mindestens ein Leitungssystem, zur Bereitstellung von Reinigungsflüssigkeit an das Düsensystem. Dabei können beispielsweise ein Düsensystem und ein Leitungssystem zur Beaufschlagung mit Reinigungsflüssigkeit aus einem Tank vorgesehen sein, sowie mindestens eine entsprechende Pumpe. Alternativ oder zusätzlich kann auch beispielsweise mindestens ein Düsensystem direkt aus einer Zuleitung beaufschlagt werden, ohne dass hierfür eine Pumpe erforderlich wäre. In der Reinigungsvorrichtung können eine oder mehrere Reinigungszonen vorgesehen sein, welche beispielsweise von dem Reinigungsgut sequenziell durchlaufen werden, beispielsweise kontinuierlich oder auch diskontinuierlich. So kann das Reinigungsgut nacheinander durch mehrere Reinigungszonen transportiert werden, in welchen eine unterschiedliche Art der Beaufschlagung mit Reinigungsflüssigkeit erfolgt, beispielsweise eine oder mehrere Reinigungszonen ausgewählt aus der Gruppe bestehend aus: einer Vorabräumzone; einer Waschzone; einer Nachspül- oder Klarspülzone, wobei selbige nochmals unterteilt sein kann in eine Pumpenklarspülzone und eine nachgelagerte Frischwasserklarspülzone. Weiterhin kann mindestens ein Trocknungsschritt vorgesehen sein, welcher beispielsweise in der einen Kammer bei stationärer Aufnahme des Reinigungsguts der Beaufschlagung mit der Reinigungsflüssigkeit nachgelagert sein kann oder welcher beispielsweise bei der Transportspülmaschine in einer den Flüssigkeits-Reinigungszonen nachgelagerten Trocknungszone erfolgen kann.

Der Begriff "Reinigungsfluid", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf ein Gas und/oder eine Flüssigkeit beziehen, welche, bei Auftreffen auf das Reinigungsgut, mindestens eine Wirkung entfalten kann, welche ausgewählt sein kann aus der Gruppe bestehend aus einer Reinigungswirkung, einer Desinfektionswirkung und einer Trocknungswirkung. Insbesondere kann das Reinigungsfluid mindestens eine wässrige Flüssigkeit umfassen, beispielsweise Wasser und/oder Wasser mit einem oder mehreren Zusatzstoffen, beispielsweise mit einem oder mehreren Reinigerkonzentraten und/oder Klarspülern und/oder Desinfektionsmitteln. Die Reinigungsvorrichtung kann eingerichtet sein, um ein einzelnes Reinigungsfluid zu verwenden oder auch um eine Kombination mehrerer Reinigungsfluide einzusetzen. Sind mehrere Reinigungsfluide vorgesehen, so kann die Beaufschlagung des Reinigungsguts mit den unterschiedlichen Reinigungsfluiden gleichzeitig oder auch sequenziell erfolgen. So kann das Reinigungsgut beispielsweise sequenziell durch mehrere Reinigungszonen transportiert werden, in denen beispielsweise eine Beaufschlagung mit unterschiedlichen Arten von Reinigungsfluiden und/oder mit Reinigungsfluiden unterschiedlicher Reinheitsgrade erfolgt. Unterschiedliche Reinheitsgrade können beispielsweise durch einen Kaskadenüberlauf zwischen verschiedenen Tanks der Transportspülmaschine erzeugt werden, wobei vorzugsweise ein Reinheitsgrad in Transportrichtung zunimmt.

Der Begriff "Transportvorrichtung", wie er hier verwendet wird, ist ebenfalls ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine Vorrichtung beziehen, welche eingerichtet ist, um mindestens eine andere Vorrichtung oder mindestens ein anderes Element, insbesondere Reinigungsgut, zu transportieren und/oder eine Bewegung der anderen Vorrichtung oder des anderen Elements anzutreiben. Die Transportvorrichtung kann insbesondere mindestens ein Antriebselement umfassen, beispielsweise mindestens ein im Kreislauf durch die Reinigungskammer laufendes Antriebselement. Insbesondere kann die Transportvorrichtung, beispielsweise das Antriebselement, mindestens ein Element umfassen, ausgewählt aus der Gruppe bestehend aus: einem Transportband, Transportrollen, insbesondere angetriebenen Transportrollen; einer Gliederkette; einem Riemenförderer; einem Klinkentransportsystem. Der Transport kann dabei beispielsweise kontinuierlich oder auch diskontinuierlich oder getaktet erfolgen, so dass beispielsweise kontinuierlich arbeitende Transportspülmaschinen oder auch getaktete Transportspülmaschinen realisiert werden können. Die Transportrichtung kann beispielsweise eine Hauptrichtung einer Bewegung des Reinigungsguts innerhalb einer Reinigungskammer der Transportspülmaschine sein. Die Transportrichtung kann fest vorgegeben sein oder kann sich auch ändern, beispielsweise lokal oder auch zeitlich. Beispielsweise kann die Transportrichtung von einem Einlauf der Transportspülmaschine hin zu einem Auslauf gerichtet sein.

Wie oben ausgeführt, ist das Reinigungsgut derart auf dem Reinigungsgut-Träger aufnehmbar, dass der Hohlraum des Reinigungsguts mit der Austrittsdüse des Reinigungsgut-Trägers in Verbindung steht. So kann der Reinigungsgut-Träger insbesondere derart ausgestaltet sein, dass das Reinigungsgut mit einer Öffnung des Hohlraums, beispielsweise einer Eintrittsöffnung eines Luftkanals, vor der Austrittsdüse positionierbar ist. Die Verbindung zwischen der Austrittsdüse des Reinigungsgut-Trägers und dem Hohlraum kann insbesondere eine fluidische Verbindung sein, so dass Reinigungsfluid von der Austrittsdüse in den Hohlraum gespritzt werden kann. Dabei kann ein direkter Kontakt zwischen dem Düsenrohr des Reinigungsgut-Trägers und dem Reinigungsgut bestehen, oder es kann ein Spalt zwischen der Austrittsdüse und dem Reinigungsgut bestehen, so dass das Reinigungsfluid zwischen der Austrittsdüse und dem Hohlraum zunächst beispielsweise eine Luftstrecke passiert. Das Reinigungsgut kann auf dem Reinigungsgut-Träger fixiert werden, beispielsweise durch eine kraftschlüssige und/oder eine formschlüssige Fixierung, oder das Reinigungsgut kann auch lediglich auf den Reinigungsgut-Träger aufgesetzt werden. Verschiedene Ausgestaltungen sind möglich.

Der Reinigungsgut-Träger ist mittels der Transportvorrichtung durch die Reinigungskammer transportierbar. Dies kann, wie oben ausgeführt, beispielsweise dadurch erfolgen, dass der Reinigungsgut-Träger in die Transportvorrichtung integriert wird, beispielsweise indem der Reinigungsgut-Träger in eine Gliederkette der Transportvorrichtung integriert wird. Alternativ kann der Reinigungsgut-Träger jedoch auch beispielsweise auf die Transportvorrichtung aufgesetzt werden, beispielsweise auf ein Transportband.

Die Beaufschlagungsvorrichtung weist mindestens eine stationäre Wurfdüse auf. Der Begriff "Wurfdüse", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine Düse beziehen, welche eingerichtet ist, um mindestens ein fluides Medium auszuwerfen, beispielsweise zu verspritzen. Die Wurfdüse kann dementsprechend insbesondere mit dem Düsenrohr zusammenwirken, welches dann als "Fangdüse" wirken kann und das von der Wurfdüse ausgegebene fluide Medium vollständig oder teilweise auffangen kann. Die Wurfdüse ist dabei stationär ausgebildet. Unter einer stationären Ausbildung kann insbesondere eine Ausbildung verstanden werden, bei welcher die Wurfdüse in einem Bezugssystem der Transportspülmaschine oder in einem Bezugssystem, in welchem die Transportspülmaschine ruht, ortsfest angeordnet ist, beispielsweise indem diese fest mit einem Gehäuse der Transportspülmaschine verbunden ist.

Diese mindestens eine stationäre Wurfdüse ist in mindestens einer Stellung des Reinigungsgut-Trägers derart relativ zu dem Reinigungsgut-Träger positioniert, dass aus der Wurfdüse austretendes Reinigungsfluid in den Fangtrichter des Düsenrohrs eintritt. So kann beispielsweise sich der Reinigungsgut-Träger aufgrund des Transports durch die Transportvorrichtung kontinuierlich oder diskontinuierlich relativ zu der Wurfdüse bewegen, so dass sich auch, das als Fangdüse wirkende, mindestens eine Düsenrohr des Reinigungsgut-Trägers relativ zu der Wurfdüse bewegt. In mindestens einer Stellung des Reinigungsgut-Trägers, bei dessen Transport mittels der Transportvorrichtung durch die Reinigungskammer hindurch, sind die Fangdüse und die Wurfdüse jedoch so zueinander positioniert, dass das aus der Wurfdüse austretende Reinigungsfluid vollständig oder teilweise in den Fangtrichter des Düsenrohres eintritt, beispielsweise indem das von der Wurfdüse verspritzte Reinigungsfluid vollständig oder teilweise von dem Fangtrichter des Düsenrohres eingefangen wird. Dies kann beispielsweise, wenn sich die Eintrittsöffnung auf der Unterseite des Basiselements befindet, dann geschehen, wenn die Wurfdüse mindestens einen nach oben gerichteten Strahl des Reinigungsfluids erzeugt und sich die Eintrittsöffnung genau über der Wurfdüse befindet. Alternativ sind entsprechende Stellungen bei einer seitlichen Anordnung der Eintrittsöffnung und/oder bei einer Anordnung auf der Oberseite des Reinigungsgut-Trägers möglich, wenn die Wurfdüse entsprechend ausgerichtet ist. Sobald der Fangtrichter an der Wurfdüse vorbeifährt, kann dann allgemein Reinigungsfluid von dem Fangtrichter aufgefangen werden, wobei das Reinigungsfluid dann vorzugsweise noch ausreichend Impuls aufweist, um das Düsenrohr zu durchdringen und mit ausreichendem Impuls durch die Austrittsdüse auszutreten, um dann in den Hohlraum des Reinigungsguts einzutreten und diesen zu reinigen. Das Reinigungsfluid kann dann also, ausgehend von der Wurfdüse, zunächst das Düsenrohr durchdringen und anschließend in den Hohlraum eintreten.

Die Transportspülmaschine kann eine oder mehrere Wurfdüsen aufweisen. Sind mehrere Wurfdüsen vorgesehen, so können diese parallel und/oder sequenziell angeordnet sein, also beispielsweise an derselben Stelle entlang eines Transportwegs oder auch an unterschiedlichen Stellen entlang dieses Transportwegs. Insbesondere können mindestens zwei der Wurfdüsen sequenziell mit dem Fangtrichter in Wechselwirkung treten. So können beispielsweise sequenziell unterschiedliche Reinigungsfluide mittels der Wurfdüsen in denselben Fangtrichter eingespritzt werden, beispielsweise zunächst ein Vorabräumfluid, anschließend ein oder mehrere Waschfluide und anschließend ein oder mehrere Klarspülfluide. Weiterhin kann sequenziell anschließend mindestens ein Trocknungsfluid eingespritzt werden, beispielsweise Trocknungsluft.

Allgemein kann die mindestens eine Wurfdüse variabel ausgestaltet sein, insbesondere hinsichtlich ihrer Strahlrichtung und/oder hinsichtlich ihrer Strahlform. Insbesondere kann das Reinigungssystem eingerichtet sein, beispielsweise mittels mindestens einer Steuerungg, um die Wurfdüse automatisch zu variieren.

Allgemein kann also die mindestens eine Wurfdüse als starre Wurfdüse ausgestaltet sein, beispielsweise mit mindestens einer fest vorgegebenen Strahlrichtung und/oder Strahlform. Alternativ oder zusätzlich kann die mindestens eine Wurfdüse jedoch auch variabel ausgestaltet sein, beispielsweise hinsichtlich der mindestens einen Strahlrichtung und/oder hinsichtlich der Strahlform. So kann die mindestens eine Wurfdüse oder, falls mehrere Wurfdüsen vorgesehen sind, mindestens eine der Wurfdüsen auch eingerichtet sein, um eine Strahlrichtung zu variieren, beispielsweise automatisch. So kann die mindestens eine Wurfdüse beispielsweise ortsfest aber schwenkbar in der Transportspülmaschine gelagert sein, so dass diese beispielsweise ihre Strahlrichtung ändern kann. Die Strahlrichtung kann beispielsweise durch einen Motor und/oder auch durch eine Hebelkonstruktion eingestellt werden. So kann die Wurfdüse beispielsweise eingerichtet sein, um den Fangtrichter zumindest über eine vorgegebene Laufstrecke des Reinigungsgut-Trägers mit dem austretenden Strahl zu verfolgen. Wiederum alternativ oder zusätzlich kann auch eine Strahlform der mindestens einen Wurfdüse oder, falls mehrere Wurfdüsen vorgesehen sind, mindestens einer der Wurfdüsen, variabel sein. So kann beispielsweise, wenn sich ein Reinigungsgut-Träger der Wurfdüse nähert, eine weite Strahlform eingestellt werden. Wenn sich der Fangtrichter dann oberhalb der Wurfdüse befindet, kann eine enge Strahlform gewählt werden, um den Fangtrichter4 gezielt zu beaufschlagen. Wenn sich der Reinigungsgut-Träger dann wieder entfernt, kann die Strahlform auch wieder aufgeweitet werden. Auch diese Einstellung der Strahlform kann automatisch erfolgen. Die Einstellung der Strahlform kann beispielsweise über eine variable Strahlblende an der Wurfdüse erfolgen, oder auch beispielsweise über einen Wechsel der Düsenblende oder Piezoelemente.

Die Beaufschlagungsvorrichtung der Transportspülmaschine kann, zusätzlich zu der mindestens einen Wurfdüse, weiterhin mindestens eine weitere Düse aufweisen. Diese mindestens eine weitere Düse kann beispielsweise derart angeordnet und/oder ausgestaltet sein, dass diese nicht mit dem Fangtrichter zusammenwirkt. So kann diese Wurfdüse beispielsweise mindestens eine äußere Oberfläche des Reinigungsguts mit Reinigungsfluid beaufschlagen. Auf diese Weise kann beispielsweise gleichzeitig oder auch zeitversetzt eine Beaufschlagung des Hohlraums und eine Beaufschlagung der mindestens einen äußeren Oberfläche des Reinigungsguts mit Reinigungsfluid erfolgen. Die mindestens eine weitere Düse kann beispielsweise als Sprühdüse und/oder als Düsenarm ausgestaltet sein. So können beispielsweise, zusätzlich zu der mindestens einen Wurfdüse, oberhalb und/oder unterhalb des Reinigungsguts jeweils ein oder mehrere Düsenarme vorgesehen sein.

Der Reinigungsgut-Träger kann eine einzelne Eintrittsöffnung aufweisen oder kann auch mehrere Eintrittsöffnungen aufweisen. Wenn der Reinigungsgut-Träger mehrere Eintrittsöffnungen aufweist, so können diese mit demselben Düsenrohr in Verbindung stehen oder auch mit mehreren unterschiedlichen Düsenrohren. Ist eine Mehrzahl an Eintrittsöffnungen vorgesehen, so kann das Reinigungssystem eingerichtet sein, um unterschiedliche Eintrittsöffnungen mit unterschiedlichen Wurfdüsen in fluidische Wechselwirkung zu bringen, insbesondere um in unterschiedlichen Behandlungsphasen unterschiedliche Eintrittsöffnungen aus unterschiedlichen Wurfdüsen mit Reinigungsfluid zu speisen. Auch andere Ausgestaltungen sind möglich.

Wie oben ausgeführt, kann die Transportspülmaschine insbesondere ausgewählt sein aus der Gruppe bestehend aus einer Bandtransportspülmaschine und einer Korbtransportspülmaschine. Dementsprechend kann die Transportvorrichtung beispielsweise mindestens ein Band und/oder mindestens eine Gliederkette und/oder mindestens einen Klinkentransport aufweisen. Auch andere Ausgestaltungen sind möglich, wie oben ausgeführt.

Die Transportspülmaschine kann insbesondere mindestens einen Sensor zur Erkennung des Reinigungsgut-Trägers aufweisen. Hierbei können beispielsweise einer oder mehrere Sensoren zum Einsatz kommen, ausgewählt aus der Gruppe bestehend aus: einem optischen Sensor; einem mechanischen Sensor; einem magnetischen Sensor; einem induktiven Sensor; einem elektromagnetischen Sensor, insbesondere einem RFID- und/oder Funksensor. Der Sensor kann direkt den Reinigungsgut-Träger erkennen und/oder kann beispielsweise auch mindestens einen Identifikator an oder in dem Reinigungsgut-Träger und/oder mindestens einen Identifikator, der sich an dem Reinigungsgut befindet, beispielsweise direkt an dem Reinigungsgut, erkennen, beispielsweise mindestens einen der oben genannten Identifikatoren.

Die Transportspülmaschine kann insbesondere mindestens eine Steuerung aufweisen. Der Begriff "teuerung", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine einteilige oder mehrteilige Vorrichtung der Reinigungsvorrichtung beziehen, welche eingerichtet ist, um einen Betrieb der Transportspülmaschine vollständig oder teilweise zu steuern und/oder zu regeln. Insbesondere kann die Steuerung eingerichtet sein, um einen oder mehrere Betriebsparameter der Transportspülmaschine zu verändern, insbesondere zu steuern und/oder zu regeln, beispielsweise eine Transportgeschwindigkeit, mindestens eine Temperatur, mindestens einen Druck, mindestens eine Pumpenleistung, mindestens eine Ventilstellung, mindestens eine Heizleistung oder auch eine Kombination zweier oder mehrerer der genannten Betriebsparameter. Die Steuerung kann insbesondere mindestens eine Datenverarbeitungsvorrichtung umfassen, beispielsweise mindestens einen Prozessor. Die Steuerung kann insbesondere programmtechnisch eingerichtet sein, beispielsweise um mindestens ein Reinigungsprogramm der Transportspülmaschine zu steuern oder durchzuführen. Dementsprechend kann die Einrichtung der Steuerung teilweise durch Hardware realisiert werden oder/auch, alternativ oder zusätzlich, vollständig oder teilweise durch Software. Weiterhin kann die Steuerung mindestens einen flüchtigen und/oder nicht flüchtigen Datenspeicher umfassen. Weiterhin kann die Steuerung mindestens eine Schnittstelle umfassen, beispielsweise eine Mensch-Maschine-Schnittstelle zur Eingabe von Befehlen und/oder zur Ausgabe von Informationen, und/oder eine drahtlose oder drahtgebundene Schnittstelle zum unidirektionalen oder bidirektionalen Austausch von Daten und/oder Befehlen zwischen der Transportspülmaschine und mindestens einer weiteren Vorrichtung. Die Steuerung kann insbesondere mindestens einen Computer und/oder mindestens einen Prozessor umfassen. Die Steuerung kann insbesondere eine zentrale oder dezentrale Maschinensteuerung der Transportspülmaschine sein.

Die Steuerung kann insbesondere eingerichtet sein, um eine Beaufschlagung der Wurfdüse mit Reinigungsfluid entsprechend der Erkennung des Reinigungsgut-Trägers zu steuern. Insbesondere kann die Steuerung eingerichtet sein, um die Beaufschlagung nur dann einzuschalten, wenn sich ein Reinigungsgut-Träger im Bereich der Wurfdüse befindet. Auf diese Weise können Ressourcen eingespart werden. Insbesondere kann die Transportspülmaschine mindestens ein Ventil und/oder mindestens einen Schalter zur Steuerung der Beaufschlagung mit dem Reinigungsfluid aufweisen.

Der mindestens eine Sensor der Transportspülmaschine kann insbesondere mindestens einen Sensor aufweisen, ausgewählt aus der Gruppe bestehend aus: einem Sensor zur Erkennung von magnetischen Kodierungen an dem Reinigungsgut-Träger; einem Sensor zur Erkennung von Indikatoren aus Metall an dem Reinigungsgut-Träger; einem Reed-Schalter; einem induktiven Näherungssensor; einem RFID-Sensor; einer Lichtschranke; einem optischen Näherungssensor; einer Bilderkennung; einem Ultraschall-Sensor; einem elektromechanischen Schalter, insbesondere einem elektromechanischen Schalter zur Zusammenwirkung mit mindestens einem Nocken an dem Reinigungsgut-Träger.

Wie oben ausgeführt, kann die Transportspülmaschine insbesondere mehrere Reinigungszonen aufweisen. Die Reinigungszonen können insbesondere von dem Reinigungsgut sequenziell durchlaufen werden. Die mindestens eine stationäre Wurfdüse kann insbesondere in mindestens einer der Reinigungszonen angeordnet sein. Die Reinigungszonen können insbesondere mindestens zwei Reinigungszonen aufweisen, ausgewählt aus der Gruppe bestehend aus: einer Vorabräumzone; einer Waschzone; einer Klarspülzone, insbesondere einer Pumpenklarspülzone und/oder einer Frischwasserklarspülzone. Weiterhin kann die Transportspülmaschine mindestens eine den Reinigungszonen nachgelagerte Trocknungszone aufweisen. In der Trocknungszone kann insbesondere mindestens eine Trocknungsdüse angeordnet sein. Die Trocknungsdüse kann in mindestens einer Stellung des Reinigungsgut-Trägers innerhalb der Trocknungszone derart relativ zu dem Reinigungsgut-Träger positioniert sein, dass aus der Trocknungsdüse austretende Trocknungsluft in den Fangtrichter des Düsenrohrs eintritt. Dabei kann derselbe Fangtrichter, welcher zuvor für flüssiges Reinigungsfluid verwendet worden ist, später auch für die Trocknung verwendet werden. Alternativ kann auch mindestens ein anderer Fangtrichter vorgesehen sein.

Wie oben ausgeführt, wird das Reinigungssystem insbesondere für die Reinigung von persönlicher Schutzausrüstung verwendet. Bei der Reinigung derartiger Güter können insbesondere giftige Abfallstoffe in das Reinigungsfluid gelangen. Dementsprechend kann die Transportspülmaschine insbesondere ein geschlossenes Tanksystem aufweisen, welches vorzugsweise nicht an die Kanalisation oder den allgemeinen Abfluss angeschlossen ist. So kann das geschlossene Tanksystem insbesondere an mindestens einen Entsorgungstank zur Entsorgung von Sondermüll angeschlossen sein.

Alternativ oder zusätzlich kann das Reinigungssystem, insbesondere die Transportspülmaschine, auch mindestens eine Aufbereitungseinrichtung aufweisen und eingerichtet sein, um das Reinigungsfluid aufzubereiten, insbesondere aufzureinigen. So kann das Reinigungssystem, insbesondere die Transportspülmaschine, mit einer oder mehreren speziellen Aufbereitungseinrichtungen ausgestattet sein, die das Reinigungsfluid schon innerhalb der Transportspülmaschine oder sogar innerhalb einer Behandlungszone der Transportspülmaschine von unerwünschten, insbesondere giftigen, Abfallstoffen befreien, beispielsweise spezielle Filtersysteme, die vorzugsweise über die Wirkung der in den Spülmaschinen bekannten Filtersysteme für Schwebstoffe hinausgehen. Beispielsweise kann mindestens eine Aufbereitungseinrichtung in Form eines Feinfilters oder Feinstfilters, beispielsweise in Form eines Tuchfilters, vorgesehen sein. Auch andere Ausgestaltungen sind möglich.

Das Reinigungssystem kann neben der mindestens einen Wurfdüse weitere Düsen umfassen. So kann die Beaufschlagungsvorrichtung beispielsweise die mindestens eine Wurfdüse sowie mindestens eine weitere Düse umfassen, wobei die mindestens eine weitere Düse beispielsweise zur Reinigung von anderen Bereichen des Reinigungsguts als dem Hohlraum eingesetzt werden kann. Dementsprechend können die Wurfdüse und die mindestens eine weitere Düse auch mit unterschiedlichen Arten von Reinigungsfluid beaufschlagt werden. Insbesondere kann der mindestens eine Hohlraum mit einer höheren Qualität an Reinigungsfluid beaufschlagt werden als beispielsweise ein äußerer Bereich des Reinigungsguts. So kann beispielsweise eine Verschleppung von Kontaminationen von mindestens einer äußeren Oberfläche persönlicher Schutzausrüstung in Bereiche, welche zur Führung von Atemgas eingesetzt werden, verhindert werden, indem beide Reinigungsfluide getrennt eingesetzt werden. So kann auch beispielsweise die mindestens eine weitere Düse im Umwälzbetrieb eingesetzt werden, wohingegen die Wurfdüse beispielsweise lediglich im einfachen Durchlauf mit frischem Reinigungsfluid beaufschlagt werden kann. Die Transportspülmaschine kann insbesondere mindestens eine Filteranlage zum Filtern von Giftstoffen aus dem Reinigungsfluid nach der Beaufschlagung und/oder vor einer erneuten Beaufschlagung des Reinigungsguts aufweisen.

Die Wurfdüse oder, wenn mehrere Wurfdüsen vorgesehen sind, mindestens eine der Wurfdüsen, kann insbesondere nach oben weisend unterhalb der Transportvorrichtung angeordnet sein. Der Fangtrichter des Düsenrohrs kann insbesondere nach unten weisend ausgerichtet sein. Das aus der Wurfdüse austretende Reinigungsfluid kann in den Fangtrichter des Düsenrohrs eintreten, wenn der Fangtrichter und die Wurfdüse zur Deckung gelangen. Wie oben ausgeführt, sind jedoch auch anders orientierte Wurfdüsen alternativ oder zusätzlich möglich, beispielsweise Wurfdüsen, welche eine Eintrittsöffnung eines Fangtrichters an einer Seitenwand des Reinigungsgut-Trägers beaufschlagen, und/oder Wurfdüsen, welche eine nach oben gerichtete Eintrittsöffnung eines Fangtrichters des Reinigungsgut-Trägers beaufschlagen.

Die Wurfdüse und das Düsenrohr können insbesondere eingerichtet sein, um einander berührungsfrei zu passieren. So kann beispielsweise ein minimaler Abstand zwischen der Wurfdüse und dem Düsenrohr 0,5 mm bis 5 cm betragen. Eine Verbindung in Form eines Anschlusses der Fluidversorgung an das Düsenrohr ist somit nicht erforderlich.

Wie oben ausgeführt, bestehen verschiedene Möglichkeiten für die Ausgestaltung der Transportvorrichtung. So kann die Transportvorrichtung insbesondere ausgewählt sein aus der Gruppe bestehend aus: einem Transportband, wobei der Reinigungsgut-Träger auf das Transportband aufgesetzt wird, insbesondere als korbförmiger Reinigungsgut-Träger; einem Transportband, wobei der Reinigungsgut-Träger mit dem Transportband verbunden wird, insbesondere verrastet wird, insbesondere als korbförmiger Reinigungsgut-Träger; als Gliederkette, wobei der Reinigungsgut-Träger als Kettenglied in die Gliederkette eingefügt ist; einem Klinkentransportsystem, wobei der Reinigungsgut-Träger auf Gleitelemente des Klinkentransportsystems aufgesetzt wird, insbesondere als korbförmiger Reinigungsgut-Träger.

In einem weiteren Aspekt der vorliegenden Erfindung wird ein Verfahren zur Reinigung von Reinigungsgut mit mindestens einem Hohlraum vorgeschlagen, wobei das Verfahren die Verwendung mindestens eines Reinigungssystems gemäß der vorliegenden Erfindung vorsieht, beispielsweise gemäß einer oder mehrerer der oben beschriebenen Ausgestaltungen und/oder gemäß einer oder mehrerer der nachfolgend noch näher beschriebenen Ausführungsformen. Das Verfahren umfasst die nachfolgend näher genannten Schritte. Diese Schritte können in der genannten Reihenfolge durchgeführt werden. Auch eine andere Reihenfolge ist jedoch grundsätzlich möglich. Weiterhin können zwei oder mehr der genannten Verfahrensschritte zeitlich überlappend oder gleichzeitig durchgeführt werden. Weiterhin können einer oder mehrere der genannten Verfahrensschritte einfach oder auch wiederholt durchgeführt werden. Das Verfahren kann über die genannten Schritte hinaus weitere Verfahrensschritte umfassen, welche nicht genannt sind.

Das Verfahren umfasst die folgenden Schritte:
i) Aufnehmen des Reinigungsguts auf dem Reinigungsgut-Träger, derart, dass der Hohlraum des Reinigungsguts mittels der Austrittsdüse des Reinigungsgut-Trägers mit mindestens einem Reinigungsfluid beaufschlagbar ist;
ii) Transport des Reinigungsgut-Trägers mittels der Transportvorrichtung durch die Reinigungskammer; und
iii) Beaufschlagen des Reinigungsguts mit mindestens einem Reinigungsfluid mittels der Beaufschlagungsvorrichtung, wobei in mindestens einer Stellung des Reinigungsgut-Trägers relativ zu der Wurfdüse aus der Wurfdüse austretendes Reinigungsfluid in den Fangtrichter des Düsenrohrs eintritt, durch das Düsenrohr hindurchtritt und aus der Austrittsdüse des Düsenrohrs in den Hohlraum des Reinigungsguts eintritt.

Für mögliche Ausgestaltungen und Definitionen kann auf die obige Beschreibung des Reinigungsgut-Trägers und/oder des Reinigungssystems verwiesen werden.

Der Reinigungsgut-Träger kann insbesondere außerhalb der Transportspülmaschine mit dem Reinigungsgut bestückt werden. Diese Bestückung kann beispielsweise unabhängig von dem Transport mittels der Transportvorrichtung erfolgen. Anschließend können der Reinigungsgut-Träger und/oder ein bestückter Teil desselben in bestückter Form in die Transportspülmaschine eingebracht werden.

Wie oben ausgeführt, kann der Reinigungsgut-Träger insbesondere auch mehrteilig ausgebildet sein. Die Transportvorrichtung kann insbesondere als umlaufende Transportvorrichtung ausgebildet sein, beispielsweise als umlaufendes Transportband und/oder als umlaufende Gliederkette. Der Reinigungsgut-Träger kann insbesondere mitsamt dem Reinigungsgut mittels der Transportvorrichtung durch die Reinigungskammer transportiert werden. Anschließend kann das Reinigungsgut sowie zumindest ein Teil des Reinigungsgut-Trägers von der Transportvorrichtung abgenommen werden. Mindestens ein weiterer Teil des Reinigungsgut-Trägers, insbesondere das Basiselement, können jedoch optional auf oder in der Transportvorrichtung bleiben und können mittels der Transportvorrichtung wieder zurück zu dem Einlauf transportiert werden. Diese Ausgestaltung kann insbesondere dann realisiert werden, wenn beispielsweise das Basiselement als Kettenelement einer Gliederkette ausgestaltet ist, wobei der Reinigungsgut-Träger mindestens ein abnehmbares Gestell auf dem Basiselement aufweist.

Wie oben ausgeführt, kann das Reinigungsgut insbesondere in oder an dem Reinigungsgut-Träger fixiert werden. Für diese Fixierung kann insbesondere das mindestens eine optionale Gestell vorgesehen sein. Die Fixierung kann insbesondere derart erfolgen, dass der mindestens eine Hohlraum des Reinigungsguts zu der Austrittsdüse ortsfest positioniert ist, insbesondere derart ortsfest, dass aus der Austrittsdüse austretendes Reinigungsfluid in den Hohlraum eindringen kann.

Wie oben ausgeführt, umfasst das Reinigungsgut insbesondere mindestens ein Teil persönlicher Schutzausrüstung. Insbesondere weist das Reinigungsgut mindestens einen Helm mit mindestens einem Visier auf. Dieser Helm kann beispielsweise mindestens einen Luftführungskanal als Hohlraum aufweisen. Der Helm kann insbesondere in dem Reinigungsgut-Träger fixiert werden. Weiterhin kann optional das Visier in dem Reinigungsgut-Träger in einer vorgegebenen Winkelstellung fixiert werden.

In einem weiteren Aspekt der vorliegenden Erfindung wird eine Verwendung des Reinigungsgut-Trägers und/oder des Reinigungssystems gemäß der vorliegenden Erfindung vorgeschlagen, beispielsweise gemäß einer oder mehreren der oben beschriebenen Ausgestaltungen und/oder gemäß einer oder mehreren der nachfolgend noch näher beschriebenen Ausführungsformen. Die Verwendung sieht eine Reinigung von persönlicher Schutzausrüstung mittels des Reinigungsgut-Trägers und/oder mittels des Reinigungssystems vor. Dabei ist die persönliche Schutzausrüstung ausgewählt aus der Gruppe bestehend aus: einer Atemschutzmaske; einem Helm mit mindestens einem Luftführungskanal.

Der vorgeschlagene Reinigungsgut-Träger, das vorgeschlagene Reinigungssystem, das Verfahren und die Verwendung weisen gegenüber herkömmlichen Vorrichtungen und Verfahren ähnlicher Art zahlreiche Vorteile auf.

So ist es mittels der vorliegenden Erfindung, insbesondere durch Verwendung des Reinigungsgut-Trägers mit dem mindestens einen Düsenrohr mit mindestens einem Fangtrichter möglich, eine große Menge an Reinigungsgut mit Hohlräumen zu reinigen, indem eine Transportspülmaschine zum Einsatz kommt. Dabei kann es sich beispielsweise um eine Bandtransportspülmaschine oder eine Korbtransportspülmaschine handeln. Je nach Anforderungen kann der Transport kontinuierlich erfolgen, oder auch diskontinuierlich. Dabei kann grundsätzlich ein kontinuierlicher Transport vorteilhaft sein, da dieser in der Regel eine größere Reinigungskapazität und Reinigungsleistung erzeugen kann. Es existieren jedoch Fälle, in denen ein diskontinuierlicher Transport hilfreich oder sogar erforderlich sein kann, beispielsweise um das Reinigungsgut jeweils an bestimmten Positionen innerhalb der Transportspülmaschine zu behandeln oder einer besonderen Behandlung zu unterziehen, beispielsweise einer besonders intensiven Behandlung.

Wie oben ausgeführt, handelt es sich bei dem Reinigungsgut insbesondere um Bestandteile persönlicher Schutzausrüstung. So umfasst das Reinigungsgut beispielsweise Atemschutzmasken oder Atemschutzhelme. Bei derartigem Reinigungsgut sowie auch bei anderem Reinigungsgut mit Hohlräumen ist in vielen Fällen damit zu rechnen, dass Bereiche innerhalb des Reinigungsgutes existieren, welche in der Regel nur gereinigt werden können, wenn das Reinigungsgut in definierter Position innerhalb der Reinigungskammer gehalten, platziert oder befestigt wird, was in Transportspülmaschinen in der Regel nicht der Fall ist. Bei der vorgeschlagenen Lösung mit dem Fangtrichter an dem Reinigungsgut-Träger und mit der Wurfdüse an der Transportspülmaschine kann eine derartige Fixierung des Reinigungsguts relativ zu dem Reinigungsgut-Träger erfolgen, welcher dann seinerseits definiert durch die Reinigungskammer transportiert werden kann. Eine Fixierung des Reinigungsguts, beispielsweise des Helms, kann beispielsweise so erfolgen, dass der Hohlraum in Überdeckung mit der Austrittsdüse auf dem Reinigungsgut-Träger kommt. Ein fester Anschluss von Leitungen für die Fluidzufuhr nicht erforderlich. Die Beaufschlagung kann während der Fahrt des Reinigungsguts durch die Reinigungskammer hindurch und somit in Bewegung erfolgen.

Weiterhin lässt sich auch die Herausforderung lösen, dass insbesondere persönliche Schutzausrüstung einige Besonderheiten bei der Reinigung aufweist. So besitzen Helme in der Regel ein bewegliches Visier, welches in der Regel während der Behandlung in einer definierten Lage gehalten werden muss. Weiter können am Helm oder an der Maske Atemluftschläuche oder Öffnungen zur Versorgung mit Atemluft vorhanden sein. Diese Öffnungen müssen in der Regel während der Reinigung verschlossen werden. Zusätzlich können am Helm oder an der Maske elektrische Anschlüsse vorhanden sein, beispielsweise zur Stromversorgung oder Steuerung eines elektrischen Gebläses. Diese Anschlüsse müssen ebenfalls in der Regel während der Reinigung ebenfalls abgedeckt sein, um einen korrosiven Angriff durch Reinigungschemikalien zu vermeiden.

Der vorgeschlagene Reinigungsgut-Träger kann diese Anforderungen einzeln oder in Kombination erfüllen. So kann dieser insbesondere separat von der Transportvorrichtung ausgestaltet sein und kann zur Reinigung in die Transportspülmaschine eingebracht werden. Dementsprechend kann der Reinigungsgut-Träger flexibel an die Art des Reinigungsguts angepasst sein, und es kann beispielsweise ein Set an verschiedenen Reinigungsgut-Trägern vorgesehen sein, welches problemlos und je nach Bedarf in die Transportspülmaschine eingebracht werden kann. Der Reinigungsgut-Träger kann insbesondere derart ausgestaltet sein, dass das Reinigungsgut, beispielsweise die Maske oder der Helm, vor der Reinigung auf diesem Reinigungsgut-Träger befestigt werden kann. Das Reinigungsgut kann beispielsweise während der gesamten Reinigung auf dem Reinigungsgut-Träger verbleiben und beispielsweise erst nach vollständiger Behandlung, insbesondere nach Durchlaufen der Reinigungskammer, von dem Reinigungsgut-Träger abgenommen werden. Verschlusselemente für die zu verschließenden Öffnungen können optional an dem Reinigungsgut-Träger befestigt sein oder in diesen integriert sein. Derartige zusätzliche Elemente können je nach Ausführung auch selbsttätig in Funktion kommen, beispielsweise indem beim Einspannen des Reinigungsguts in den Reinigungsgut-Träger selbsttätig mindestens eine zu verschließende Öffnung und/oder ein elektrischer Kontakt verschlossen werden.

Der Reinigungsgut-Träger kann fest mit der Transportvorrichtung der Transportspülmaschine, beispielsweise einem Transportband, verbunden sein. Dementsprechend kann dieser auch ganz oder teilweise selbst Bestandteil der Transportvorrichtung sein. Alternativ kann der Reinigungsgut-Träger auch beispielsweise nur auf die Transportvorrichtung aufgesetzt und/oder mit derselben verbunden werden, beispielsweise in die Transportvorrichtung eingerastet werden und am Auslauf der Transportspülmaschine wieder abgenommen werden. Letzteres Prinzip kann insbesondere analog zum Prinzip der Reinigungskörbe bei Korbtransport-Geschirrspülmaschinen ausgestaltet sein. Wenn der Reinigungsgut-Träger ganz oder teilweise abnehmbar ausgeführt ist, kann er insbesondere außerhalb der Transportspülmaschine mit dem Reinigungsgut bestückt werden und dann voll bestückt in die Transportspülmaschine eingebracht werden.

Je nach Aufbauhöhe des Reinigungsgut-Trägers kann es, besonders bei einer Bandtransportspülmaschine, sinnvoll sein, dass der gesamte oder auch nur Teile des Reinigungsgut-Trägers am Einlauf auf die Transportvorrichtung, insbesondere das Transportband, aufgesetzt und am Auslauf der Transportspülmaschine wieder abgenommen werden. Auf diese Weise kann beispielsweise nach einer Umlenkung das zurücklaufende Transportband ungehindert laufen, ohne dass der zurücklaufende Reinigungsgut-Träger unnötig viel Bauraum beanspruchen würde.

Der Reinigungsgut-Träger kann weiterhin insbesondere so ausgeführt sein, dass er jeweils nur ein Stück Reinigungsgut aufnehmen kann, beispielsweise nur jeweils eine Maske oder einen Helm. Es ist jedoch auch möglich, den Reinigungsgut-Träger derart zu gestalten, dass mehrere Stücke an Reinigungsgut aufgenommen werden können, beispielsweise zwei oder mehr Helme. Diese können beispielsweise, in Transportrichtung der Transportspülmaschine gesehen, nebeneinander angeordnet sein. Auch andere Konstellationen sind denkbar.

Um einen größeren Durchsatz an Reinigungsgut zu erzielen, ist es insbesondere zweckmäßig, innerhalb der Transportspülmaschine mehrere Reinigungsgut-Träger gleichzeitig zu verwenden. Sind mehrere Reinigungsgut-Träger in der Transportspülmaschine vorgesehen, so können diese identisch ausgestaltet sein. Es ist jedoch auch möglich, innerhalb der Transportspülmaschine und in dem Reinigungssystem mehrere unterschiedliche Arten an Reinigungsgut-Trägern vorzusehen, beispielsweise Reinigungsgut-Träger für unterschiedliche Arten von Reinigungsgut.

Allgemein kann der mindestens eine Reinigungsgut-Träger mindestens eine Schnittstelle aufweisen, um mit der Transportvorrichtung zu interagieren, insbesondere eine Schnittstelle, welche eingerichtet ist, um an die Transportvorrichtung zu koppeln. Hierbei kann es sich beispielsweise um eine kraftschlüssige und/oder formschlüssige Kopplung handeln. Insbesondere kann der Reinigungsgut-Träger in die Transportvorrichtung eingehakt oder eingerastet werden. Dementsprechend können die Transportvorrichtung und/oder der Reinigungsgut-Träger, insbesondere die Schnittstelle, mindestens ein Verbindungselement aufweisen, welches eingerichtet ist, um den Reinigungsgut-Träger an die Transportvorrichtung zu koppeln, insbesondere reversibel. Diese Schnittstelle kann insbesondere für die Reinigungsgut-Träger universell ausgestaltet sein. Sind mehrere Reinigungsgut-Träger vorgesehen, so können diese beispielsweise über dieselbe Schnittstelle verfügen. Auch wenn mehrere Arten von Reinigungsgut-Trägern in dem Reinigungssystem vorgesehen sind, so können diese beispielsweise über dieselbe Schnittstelle zur Kopplung an die Transportvorrichtung verfügen. So kann allgemein eine Geometrie der Reinigungsgut-Träger, welche mit der Transportvorrichtung der Transportspülmaschine interagiert, bei jedem Reinigungsgut-Träger identisch sein. Darüber hinaus kann der Reinigungsgut-Träger, neben der Schnittstelle und deren Geometrie, mindestens eine weitere Geometrie aufweisen, welche von Reinigungsgut-Träger zu Reinigungsgut-Träger unterschiedlich ausgestaltet sein kann. So kann diese weitere Geometrie, welche auch als Hauptgeometrie bezeichnet werden kann, ausgestaltet sein, um das Reinigungsgut aufzunehmen und beispielsweise zu fixieren. Diese Hauptgeometrie kann jeweils individuell unterschiedlich je nach Anforderung des Reinigungsguts ausgestaltet sein.

Es ist somit möglich, viele unterschiedliche Arten von Reinigungsgut-Trägern in dem Reinigungssystem einzusetzen. Um individuell, kostengünstig und effizient, unterschiedliche Arten von Reinigungsgut-Trägern herzustellen, können diese Reinigungsgut-Träger oder Teile derselben, beispielsweise die Teile, welche die Hauptgeometrie zur Fixierung des Reinigungsguts beinhalten, mit Herstellungsverfahren gestaltet werden, welche schnell in ihrer Geometrie veränderlich sind. So kann beispielsweise der Reinigungsgut-Träger ganz oder teilweise aus mindestens einem additiven Fertigungsverfahren hergestellt sein, beispielsweise Kunststoff-Lasersintem.

Das mindestens eine Düsenrohr kann allgemein als mindestens ein fluidführendes Element ausgestaltet sein und kann eine einfache oder auch eine komplexe Kanalgeometrie bereitstellen. So kann das mindestens eine Düsenrohr ein oder mehrere oder mehrere fluidführende Elemente aufweisen, welche in den Reinigungsgut-Träger, insbesondere das Basiselement, integriert und/oder angebaut sein können. Mit diesem mindestens einen Düsenrohr können ein oder mehrere Reinigungsfluide, beispielsweise Hauptreinigungsfluide, Klarspülfluide oder auch Luft zur Trocknung, zweckdienlich geleitet und/oder als Strahlen geformt werden. Diese fluidführenden Elemente können beispielsweise als Trichter, Düsen, Leitschaufeln oder Rohrleitungen oder auch als Kombinationen aus diesen Elementen ausgeführt sein. Weiterhin können auch ein oder mehrere fluidführende Elemente ganz oder teilweise in die Transportvorrichtung integriert sein. Ist der Reinigungsgut-Träger beispielsweise ganz oder teilweise in die Transportvorrichtung integriert, so können ein oder mehrere fluidführende Elemente auch an der Transportvorrichtung, beispielsweise am Transportband, befestigt sein oder in diese integriert sein.

Um das Düsenrohr, insbesondere das Fluidsystem in dem Reinigungsgut-Träger, mit Reinigungsfluid zu versorgen, weist das Düsenrohr den Fangtrichter auf. Dieser kann einen Fluidstrahl, der von einer Transportspülmaschinen-seitig platzierten Düse, auch als Wurfdüse bezeichnet, abgegeben wird, auffangen und durch das weitere Düsenrohr zu der Austrittsdüse leiten. Beispielsweise kann die Austrittsdüse als Spritzdüse ausgestaltet sein. Auf diese Weise ist es möglich, dass das Reinigungsgut, obwohl sich dieses in Bewegung befinden kann, einer gezielten Behandlung unterzogen werden kann, einschließlich des mindestens einen Hohlraums. Innerhalb der Transportspülmaschine können dabei mehrere Wurfdüsen vorgesehen sein. Durch entsprechende Ausgestaltung von Fangtrichter und Wurfdüsen ist es dabei in der Regel nicht erforderlich, dass die Fluidversorgung des Reinigungsgut-Trägers fluiddicht erfolgen muss. Zwischen der Wurfdüse und dem Düsenrohr muss somit keine fluiddichte Verbindung bestehen. Der Fangtrichter kann zudem derart ausgestaltet sein, dass dieser Toleranzen in der Positionierung zwischen dem Reinigungsgut-Träger und der Wurfdüse ausgleichen kann. Zudem können bewegungsbedingte Schwankungen der Positionierung ausgeglichen werden, und es kann, durch eine entsprechende geometrische Ausgestaltung des Fangtrichters, auch ein Fluidübertrag auf einer längeren Wegstrecke erfolgen.

Sind mehrere Wurfdüsen vorgesehen, so können diese beispielsweise jeweils für verschiedene Phasen der Behandlung vorgesehen sein, beispielsweise in den jeweiligen Behandlungszonen, wie beispielsweise Vorreinigen, Hauptreinigen, Zwischenspülen, Desinfizieren, Klarspülen, Ausblasen und Trocknen. Alternativ oder zusätzlich können auch pro Behandlungsschritt mehrere Wurfdüsen vorgesehen sein.

Sind mehrere Wurfdüsen in derselben Behandlungszone und/oder in derselben Behandlungsphase vorgesehen, so können diese beispielsweise sequenziell von dem Reinigungsgut-Träger passiert werden. Hierdurch kann sich ein vorteilhafter Effekt beispielsweise durch eine pulsierende Beaufschlagung oder auch durch eine längere Einwirkdauer ergeben. Alternativ oder zusätzlich können jedoch auch mehrere Wurfdüsen parallel zueinander in derselben Behandlungszone vorgesehen sein.

Der Fangtrichter kann allgemein in seiner Geometrie an die jeweiligen Erfordernisse angepasst werden. So kann die Eintrittsöffnung beispielsweise mit einer kreisrunden Form ausgestaltet sein, oder auch beispielsweise, wie oben ausgeführt, längsoval. Auch andere Formen sind möglich, je nach Erfordernissen. Insbesondere kann die Eintrittsöffnung lang gestreckt sein, beispielsweise in Transportrichtung. So kann eine einzelne Wurfdüse das Fluidsystem des Reinigungsgut-Trägers auf einer längeren Strecke mit Reinigungsfluid versorgen.

Der Fangtrichter kann, wie oben ausgeführt, an unterschiedlichen Stellen am Reinigungsgut-Träger angeordnet sein. So kann dieser an einer oder mehreren Seiten angeordnet sein, ausgewählt aus der Unterseite, der Oberseite oder einer Seitenwand, beispielsweise der Längsseite. Die entsprechenden Wurfdüsen können innerhalb der Transportspülmaschine in entsprechender Art und Weise angeordnet sein. Weiterhin können auch mehrere unterschiedliche Düsenrohre, beispielsweise in Form unterschiedlicher Fluidsysteme, an oder in dem Reinigungsgut-Träger vorgesehen sein, beispielsweise für unterschiedliche Behandlungsphasen. Diese unterschiedlichen Düsenrohre können dann beispielsweise aus unterschiedlichen Wurfdüsen der Transportspülmaschine gespeist werden.

Die mindestens eine Wurfdüse der Transportspülmaschine kann kontinuierlich mit dem jeweiligen Reinigungsfluid versorgt werden, oder die Versorgung mit dem Reinigungsfluid kann diskontinuierlich erfolgen, beispielsweise geschaltet. So kann eine Beaufschlagung der mindestens einen Wurfdüse der Transportspülmaschine insbesondere entsprechend mindestens eines Parameters geschaltet oder gesteuert erfolgen, ausgewählt aus der Gruppe bestehend aus: einer Position des Reinigungsgut-Trägers; einer Art des Reinigungsgut-Trägers; einer Art des Reinigungsguts. Alternativ oder zusätzlich sind auch andere Randbedingungen für die Schaltung oder Steuerung der Beaufschlagung der mindestens einen Wurfdüse mit Reinigungsfluid möglich. Allgemein können durch diese Steuerung der Beaufschlagung der Wurfdüse mit Reinigungsfluid beispielsweise spezielle Behandlungsabfolgen realisiert werden, und/oder Ressourcen können eingespart werden, indem beispielsweise bestimmte Reinigungsfluide nur verspritzt werden, wenn diese auch benötigt werden. Die Schaltung der Wurfdüsen kann beispielsweise mittels elektromotorischen Ventilen und/oder elektromagnetischen Ventilen erfolgen. Auch rein mechanische Lösungen sind möglich, bei denen beispielsweise der Reinigungsgut-Träger selbst, mit einem Betätigungselement, beispielsweise einem Schleifbügel, zusammenwirkt und beispielsweise eine Klappe oder einen Schieber an der Wurfdüse öffnet. Verschiedene Ausgestaltungen einer gesteuerten Beaufschlagung der mindestens einen Wurfdüse mit Reinigungsfluid sind möglich.

Die mindestens eine Austrittsdüse des Reinigungsgut-Trägers, beispielsweise die Spritzdüse, kann je nach Reinigungsgut individuell ausgebildet sein. So kann die Ausgestaltung der mindestens einen Austrittsdüse insbesondere derart erfolgen, dass das von dieser Austrittsdüse abgegebene Reinigungsfluid mit möglichst maximaler Wirkung auf das Reinigungsgut aufgebracht wird. Innerhalb des Düsenrohrs und/oder am Auslass der Austrittsdüse kann auch eine innere Struktur eingebracht sein, um die Strömung des Reinigungsfluids zu beeinflussen und/oder um die Wirkung der Austrittsdüse zu optimieren. So kann beispielsweise eine Geometrie vorgesehen sein, welche einen Drall im Fluidstrahl des Reinigungsfluids erzeugt, und/oder eine gitterartige Struktur.

Je nach Anforderungen an den Reinigungsprozess kann es weiterhin hilfreich oder notwendig sein, dass bekannt ist, an welcher Stelle innerhalb der Transportspülmaschine sich das Reinigungsgut, beispielsweise eine Maske, und/oder der Reinigungsgut-Träger befindet. Zur Erkennung und/oder Bestimmung der Position des Reinigungsguts und/oder des mindestens einen Reinigungsgut-Trägers in der Transportspülmaschine können unterschiedliche Methoden zum Einsatz kommen. So können beispielsweise magnetische Kodierungen an den Reinigungsgut-Trägern mit entsprechenden Sensoren in der Transportspülmaschine erfasst werden. Alternativ oder zusätzlich können auch Indikatoren aus Metall und/oder magnetische Indikatoren an den Reinigungsgut-Trägern zum Einsatz kommen, sowie entsprechende Sensoren. Wiederum alternativ oder zusätzlich können induktive Näherungssensoren, RFID-Technik, Lichtschranken, optische Näherungssensoren, Technologien mit optischer Bilderkennung, Technologien mit Ultraschall-Sensorik, Nocken und zugehörige Schalter oder andere Techniken zur Erkennung des Reinigungsguts und/oder des mindestens einen Reinigungsgut-Trägers zum Einsatz kommen.

Wie oben ausgeführt, wird mittels des vorgeschlagenen Reinigungsgut-Trägers und des vorgeschlagenen Reinigungssystems insbesondere persönliche Schutzausrüstung gereinigt. Insbesondere dabei, jedoch auch bei anderem Reinigungsgut, können Schadstoffe freigesetzt werden, welche beispielsweise mit dem Reinigungsfluid in mindestens einen Tank der Transportspülmaschine gespült werden. Dabei können innerhalb der Transportspülmaschine beispielsweise mehrere Tanks vorgesehen sein, welche zum Beispiel mittels eines Kaskadensystems in Reihe geschaltet sind, so dass beispielsweise das Reinigungsgut zunächst eine oder mehrere Reinigungszonen mit niedrigem Reinheitsgrad durchläuft, bevor eine oder mehrere Reinigungszonen mit einem höheren Reinheitsgrad, beispielsweise einer geringeren Schadstoffkonzentration, durchlaufen werden. So kann beispielsweise ein Waschtank ausreichend getrennt von mindestens einem nachfolgenden Klarspültank getrennt ausgebildet sein, so dass mindestens ein Klarspültank, aus welchem beispielsweise mindestens eine Pumpenklarspülung gespeist wird, nicht mit Schadstoffen aus dem Waschtank beaufschlagt wird. Weiterhin können, alternativ oder zusätzlich, zwei oder mehr Pumpenklarspülungen in Reihe geschaltet werden, wobei beispielsweise die Klarspültanks dieser Pumpenklarspülungen mit einer entsprechenden Filtertechnik ausgerüstet sein können.

Mittels des vorgeschlagenen Reinigungsgut-Trägers und des vorgeschlagenen Reinigungssystems lässt sich insgesamt der apparative und personelle Aufwand für die Reinigung von Reinigungsgut mit mindestens einem Hohlraum bei hohem Durchsatz im Vergleich zu herkömmlichen Systemen deutlich verbessern. So muss in der Regel beispielsweise für die Reinigung von Masken lediglich eine Spülmaschine vorgesehen werden, welche als Transportspülmaschine ausgestaltet ist. Mit dieser Transportspülmaschine kann das gesamte Aufkommen an Reinigungsgut behandelt werden.

Weiterhin kann der Betrieb des Reinigungssystems deutlich ökonomischer gestaltet werden als bei herkömmlichen Systemen. Insbesondere kann die Verwendung der Transportspülmaschine im Vergleich zur Verwendung mehrerer einzelner Einkammer-Spülmaschinen deutlich ökonomischer gestaltet werden. Dabei lässt sich ein effizienter und ergonomischer Materialfluss einrichten, und ein effizienter Einsatz von Ressourcen wird ermöglicht.

### Kurze Beschreibung der Figuren

Weitere Einzelheiten und Merkmale ergeben sich aus der nachfolgenden Beschreibung von Ausführungsbeispielen, insbesondere in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktionen einander entsprechende Elemente.

Im Einzelnen zeigen:
- Figur 1: ein erstes Ausführungsbeispiel eines Reinigungssystems zur Reinigung von Reinigungsgut mit mindestens einem Hohlraum in einer Schnittdarstellung von der Seite;
- Figuren 2 bis 4: verschiedene Darstellungen eines ersten Ausführungsbeispiels eines Reinigungsgut-Trägers;
- Figur 5: ein zweites Ausführungsbeispiel eines Reinigungssystems zur Reinigung von Reinigungsgut mit mindestens einem Hohlraum in einer Schnittdarstellung von der Seite;
- Figur 6: ein zweites Ausführungsbeispiel eines als Reinigungskorb ausgestalteten Reinigungsgut-Trägers in einer Schnittdarstellung von der Seite; und
- Figur 7: einen schematischen Ablaufplan eines Ausführungsbeispiels eines erfindungsgemäßen Verfahrens zur Reinigung von Reinigungsgut mit mindestens einem Hohlraum.

### Beschreibung der Ausführungsbeispiele

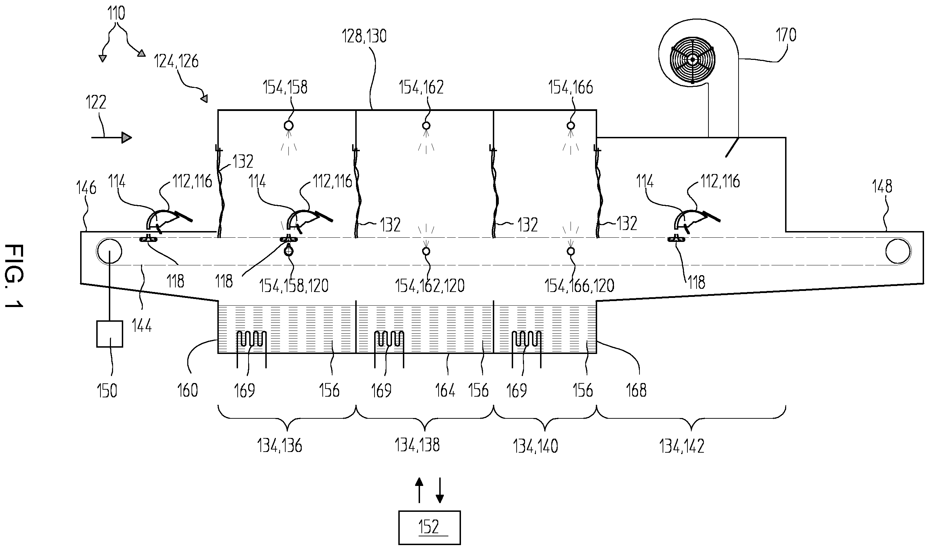

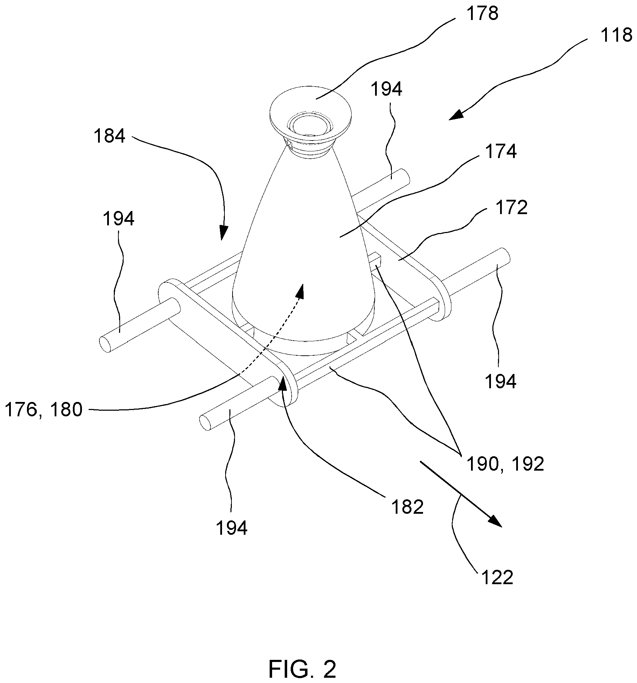

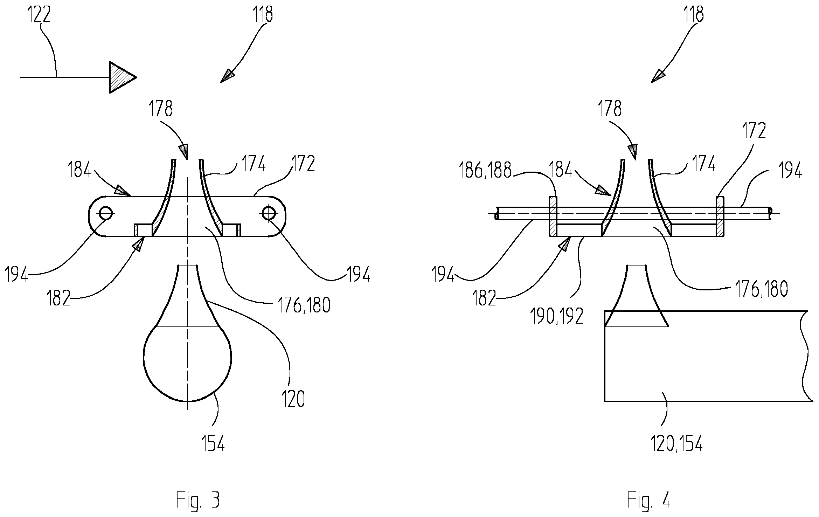

In Figur 1 ist ein erstes Ausführungsbeispiel eines Reinigungssystems 110 zur Reinigung von Reinigungsgut 112 mit mindestens einem Hohlraum 114 gezeigt. Das Reinigungsgut 112 ist exemplarisch in diesem Ausführungsbeispiel als Helm 116 einer persönlichen Schutzausrüstung dargestellt. Das Reinigungsgut 112 ist aufgenommen auf Reinigungsgut-Trägern 118, welche exemplarisch in einem ersten Ausführungsbeispiel in den Figuren 2, 3 und 4 gezeigt sind. Dabei zeigt Figur 2 eine perspektivische Darstellung eines Reinigungsgut-Trägers 118, Figur 3 zeigt eine Schnittdarstellung durch den Reinigungsgut-Träger 118 sowie eine nachfolgend noch näher erläuterte Wurfdüse 120 in einem Schnitt parallel zu einer Transportrichtung 122 des Reinigungssystems 110, und Figur 4 zeigt die Anordnung gemäß Figur 3 in einer Schnittdarstellung mit Schnittebene senkrecht zu der Transportrichtung 122. Die Figuren 1 bis 4 werden im Folgenden gemeinsam erläutert.

Das Reinigungssystem 110 umfasst in diesem Ausführungsbeispiel, neben einer Mehrzahl der Reinigungsgut-Träger 118, eine Transportspülmaschine 124. Diese Transportspülmaschine 124 ist in dem dargestellten Ausführungsbeispiel exemplarisch als Bandspülmaschine 126, auch als Bandtransport-Spülmaschine bezeichnet, ausgestaltet. Sie weist eine Reinigungskammer 128 in Form eines Reinigungstunnels 130 auf. Der Reinigungstunnel 130 kann insbesondere durch Trennvorhänge 132 in mehrere Zonen 134, auch als Reinigungszonen bezeichnet, unterteilt sein, wobei in dem dargestellten Ausführungsbeispiel exemplarisch eine Vorabräumzone 136, eine Spülzone 138, auch als Waschzone bezeichnet, eine Klarspülzone 140, auch als Nachspülzone bezeichnet, und eine Trocknungszone 142 vorgesehen sind. Die Klarspülzone 140 kann beispielsweise nochmals unterteilt sein in eine Pumpenklarspülzone und eine Frischwasserklarspülzone. Auch eine andere Ausgestaltung der Zonen 134 ist möglich.

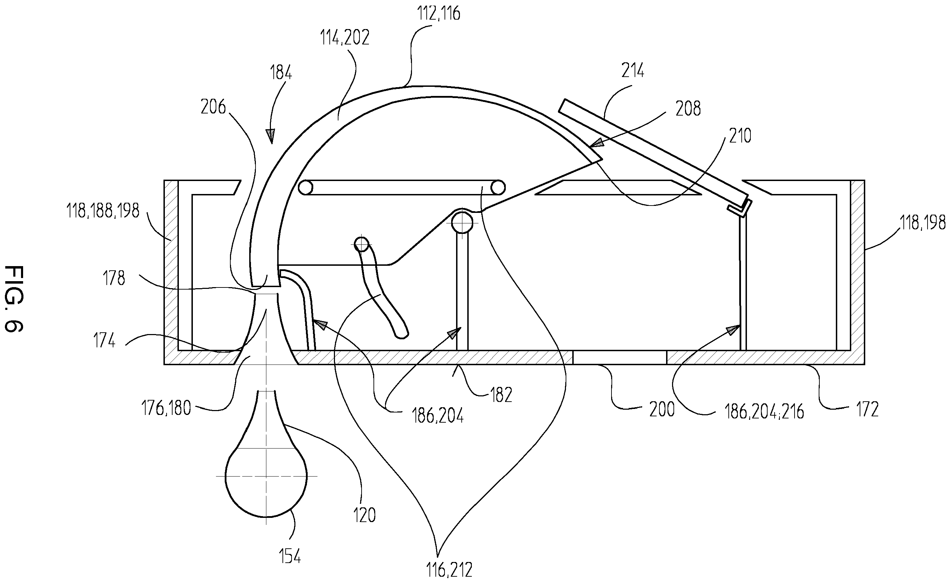

In dem dargestellten Ausführungsbeispiel wird Reinigungsgut 112, in Form von Gegenständen persönlicher Schutzausrüstung, mittels mindestens einer Transportvorrichtung 144 in der Transportrichtung 122 durch den Reinigungstunnel 130 befördert. Zu diesem Zweck kann die Transportspülmaschine 124 beispielsweise, wie in Figur 1 gezeigt, als Bandtransportmaschine 126 ausgestaltet sein, oder auch, wie nachfolgend anhand der Figur 6 noch näher gezeigt, als Korbtransportmaschine. Beispielsweise kann mindestens ein Einlauf oder Einlaufbereich 146 vorgesehen sein, an welchem das Reinigungsgut 112 auf die Transportvorrichtung 144 aufgegeben wird, und mindestens ein Auslauf oder Auslaufbereich 148, an welchem das gereinigte Reinigungsgut 112 entnommen werden kann. Die Transportvorrichtung 144 kann dementsprechend beispielsweise mindestens ein Band und/oder mindestens eine Gliederkette umfassen. Auch andere Ausgestaltungen sind möglich. Die Transportvorrichtung 144 kann beispielsweise mindestens einen Antrieb 150 umfassen, beispielsweise einen Motor, welcher beispielsweise durch eine Steuerung 152 angesteuert werden kann.

In den Zonen 136, 138 und 140 kann das Reinigungsgut 112 mittels mindestens einer Beaufschlagungsvorrichtung 154 mit mindestens einem Reinigungsfluid 156, beispielsweise mindestens einer Reinigungsflüssigkeit, beaufschlagt werden. Beispielsweise kann zu diesem Zweck in der Vorabräumzone 136 mindestens ein Vorabräumzonen-Düsensystem 158 vorgesehen sein, welches, beispielsweise über eine in Figur 1 nicht dargestellte Pumpe und ein nicht dargestelltes Leitungssystem, aus einem Vorabräumtank 160 mit Reinigungsfluid 156 gespeist werden kann. In der Spülzone 138 kann beispielsweise ein Spülzonen-Düsensystem 162 vorgesehen sein, welches, beispielsweise über eine ebenfalls nicht dargestellte Pumpe und ein ebenfalls nicht dargestelltes Leitungssystem, aus einem Spültank 164, auch als Waschtank bezeichnet, mit Reinigungsfluid 156 gespeist werden kann. In der Klarspülzone 140, welche einteilig oder auch mehrteilig ausgestaltet sein kann, kann beispielsweise mindestens ein Nachspüldüsensystem 166, auch als Klarspüldüsensystem bezeichnet, vorgesehen sein, welches mit Reinigungsfluid 156 in Form beispielsweise von erwärmtem Frischwasser und/oder mit Nachspülfluid aus einem Nachspültank 168, auch als Klarspültank bezeichnet, gespeist werden kann. In den Tanks 160, 164 und 168 können beispielsweise Heizvorrichtungen 169 vorgesehen sein. Weiterhin können in diesen Tanks 160, 164 und 168 auch Filtervorrichtungen vorgesehen sein, beispielsweise um in einem Umwälzbetrieb eine Aufreinigung des Reinigungsfluids 156 zu bewirken. Weiterhin können die Tanks 160, 164 und 168 mit einem Kaskadenüberlauf und/oder einer gepumpten Kaskade ausgestaltet sein, so dass beispielsweise in dem Nachspültank 168 ein höchster Reinheitsgrad und in dem Vorabräumtank 160 der geringste Reinheitsgrad herrscht.

Nach Durchlaufen der Zonen 136, 138 und 140 kann das Reinigungsgut 112 dann in die Trocknungszone 142 eintreten, in welcher das Reinigungsgut 112 beispielsweise über ein Gebläse 170 mit Warmluft beaufschlagt werden kann, um die Trocknung des Reinigungsguts 112 zu beschleunigen. Der Auslaufbereich 148 ist der Reinigungskammer 128 in Transportrichtung 122 nachgelagert, so dass das gereinigte Reinigungsgut 112 im Auslaufbereich 148 von der Transportvorrichtung 144 entnommen werden kann.

Bei der dargestellten Transportspülmaschine 124 besteht grundsätzlich das Prinzip, dass die Beaufschlagungsvorrichtung 154 ortsfest ausgestaltet ist, wohingegen das Reinigungsgut 112 in der Transportrichtung 122 relativ zu dieser Beaufschlagungsvorrichtung 154 bewegt wird. Dieses Reinigungsprinzip kann insbesondere bei Reinigungsgut 112 mit mindestens einem Hohlraum 114 zu Herausforderungen dahingehend führen, dass keine ausreichende Menge an Reinigungsfluid 156 in den Hohlraum 114 gelangt. Bei dem Hohlraum 114 kann es sich insbesondere um einen lang gestreckten Hohlraum handeln, beispielsweise mindestens einen Luftkanal, wie unten am Beispiel der Figur 6 noch näher erläutert wird. So passieren der Hohlraum 114 und/oder eine Eintrittsöffnung dieses Hohlraums 114 die Düsen der Beaufschlagungsvorrichtung 154 in der Regel zu schnell, als dass ausreichend Reinigungsfluid in diesen Hohlraum 114 gelangen könnte. Weiterhin ist der Hohlraum 114 in vielen Fällen durch Bestandteile der Transportvorrichtung 144 verdeckt. Zur Lösung dieser Problematik wird in dem in den Figuren 2 bis 4 dargestellten Ausführungsbeispiel vorgeschlagen, dass der Reinigungsgut-Träger 118 mindestens ein Basiselement 172 aufweist, sowie weiterhin mindestens ein mit dem Basiselement 172 verbundenes Düsenrohr 174. In dem beweglichen Reinigungsgut-Träger 118 ist also das Düsenrohr 174 integriert, welches als bewegliche Reinigungsdüse wirken kann und welches eine Kanalstruktur für eine Fluidführung bereitstellen kann. Das Düsenrohr 174 weist mindestens eine Eintrittsöffnung 176 sowie mindestens eine Austrittsdüse 178, auch als Austrittsöffnung bezeichnet, auf. Die Austrittsdüse 178 wirkt als in der Reinigungskammer 128 bewegliche, durch die Transportvorrichtung 144 transportierte Reinigungsdüse. Das Düsenrohr 174 weist an seiner Eintrittsöffnung 176 einen Fangtrichter 180 auf, so dass das Düsenrohr 174 in seinem Inneren zumindest abschnittsweise verjüngt ausgebildet ist. Insbesondere kann die Eintrittsöffnung 176 einen deutlich größeren Durchmesser oder Äquivalentdurchmesser aufweisen als die Austrittsdüse 178. Somit ist das Düsenrohr 174 so gestaltet, dass der wirksame Querschnitt auf der Seite der Eintrittsöffnung 176 für einen Einfang von Reinigungsfluid 156 deutlich größer ist als der Durchmesser oder Äquivalentdurchmesser der Austrittsdüse 178 und/oder als ein Durchmesser des mindestens einen Hohlraums 114 des Reinigungsguts 112 oder eine Eintrittsöffnung dieses Hohlraums 114. Im Vergleich zu einer Situation, in welcher die Beaufschlagungsvorrichtung 154 direkt in den Hohlraum 114 einspritzen würde, ist somit der Wirkungsquerschnitt für das Eindringen von Reinigungsfluid 156 in den Hohlraum 114 durch den Fangtrichter 180 deutlich erhöht.

Das Düsenrohr 174 ist beispielsweise in dem dargestellten Ausführungsbeispiel derart aufgenommen in dem Basiselement 172, dass die Eintrittsöffnung 176 auf einer Unterseite 182 des Basiselements 172 angeordnet ist, während die Austrittsdüse 178 auf einer Oberseite 184 des Basiselements 172 angeordnet ist. Das Basiselement 172 kann beispielsweise auf der Oberseite 184 ein Gestell 186 aufweisen, welches beispielsweise einen vollständig oder teilweise umlaufenden Rahmen 188 bildet. An diesem Rahmen 188 kann beispielsweise das Reinigungsgut 112 befestigt werden oder eingespannt werden, so dass beispielsweise mindestens eine Öffnung des Hohlraums 114 mit der Austrittsdüse 178 zur Deckung kommt. Die Beaufschlagungsvorrichtung 154 kann beispielsweise, wie in den Figuren 3 und 4 erkennbar, die mindestens eine Wurfdüse 120, welche beispielsweise Bestandteil eines unteren Düsensystems der Transportspülmaschine 124 gemäß Figur 1 sein kann, umfassen. Diese Wurfdüse 120 kann beispielsweise, wie in den Figuren 3 und 4 erkennbar, nach oben ausgerichtet sein. Sobald die Eintrittsöffnung 176 mit der mindestens einen Wurfdüse 120 zur Deckung kommt, kann aus der Wurfdüse 120 austretendes Reinigungsfluid 156 in den Fangtrichter 180 eintreten und kann aufgrund seines Impulses auf der Oberseite 184 aus der Austrittsdüse 178 austreten und in den Hohlraum 114 des Reinigungsguts 112 eingespritzt werden. Gleichzeitig oder zeitversetzt kann eine weitere Beaufschlagung von einer oder mehreren Raumrichtungen her aus anderen Düsen der Beaufschlagungsvorrichtung 154 erfolgen, so dass, zusätzlich zu dem Hohlraum 114, auch mindestens eine Oberfläche des Reinigungsguts 112 durch Reinigungsfluid 156 gereinigt werden kann.

Die Anordnung der Eintrittsöffnung 176 auf der Unterseite 182 des Basiselements 172 ist grundsätzlich zu bevorzugen, da dann Reinigungsfluid 156 nach der Beaufschlagung des Hohlraums 114 auch wieder, getrieben durch seine Schwerkraft, nach unten hin ablaufen kann. Alternativ oder zusätzlich sind jedoch auch andere Möglichkeiten denkbar. So kann die Eintrittsöffnung 176 auch an mindestens einer Seitenwand des Basiselements 172, beispielsweise an mindestens einer Längsseite, und/oder auch auf der Oberseite 184 angeordnet sein. Weiterhin können eine oder auch mehrere Eintrittsöffnungen 176 vorgesehen sein. Die Eintrittsöffnung 176 kann beispielsweise kreisrund ausgestaltet sein. Alternativ kann jedoch auch eine Ausgestaltung möglich sein, bei welcher beispielsweise eine Ausdehnung in Transportrichtung 122 länger ist als quer zur Transportrichtung 122, so dass über eine längere Wegstrecke Reinigungsfluid 156 von dem Fangtrichter 180 eingefangen wird. Hierdurch kann eine Zeitdauer der Beaufschlagung verlängert werden.

Das mindestens eine Düsenrohr 174 kann das Basiselement 172 beispielsweise durchdringen und/oder kann auf andere Weise in dem Basiselement 172 oder an dem Basiselement 172 gehalten sein. Beispielsweise kann das Basiselement 172, wie in Figur 2 erkennbar, als Gitter 190 ausgestaltet sein und kann eine Mehrzahl von Gitterstreben 192 aufweisen, welche das Düsenrohr 174 mit dem Rahmen 188 verbinden. Auf diese Weise kann Reinigungsfluid durch das Basiselement 172 nach unten ablaufen.

Wie oben ausgeführt, kann die Transportvorrichtung 144 der Transportspülmaschine 124 insbesondere als Band oder als Gliederkette ausgestaltet sein. Das Basiselement 172 kann beispielsweise in die Transportvorrichtung 144 integrierbar sein und/oder mit der Transportvorrichtung 144 verbindbar sein, vorzugsweise reversibel. So kann das Basiselement 172 beispielsweise als Kettenglied einer Gliederkette ausgebildet sein, beispielsweise mit Bohrungen, durch die Bandstangen 194 der Transportvorrichtung geführt sind, die sich dann weiter nach beiden Seiten erstrecken.

Der Reinigungsgut-Träger 118 kann einteilig oder auch mehrteilig ausgestaltet sein. So kann beispielsweise das Gestell 186 vollständig oder teilweise von dem Basiselement 172 abnehmbar sein. Beispielsweise kann das abnehmbare Teil mit dem Reinigungsgut 112 verbunden sein und kann im Auslaufbereich 148 von der Transportvorrichtung 144 abgenommen werden.

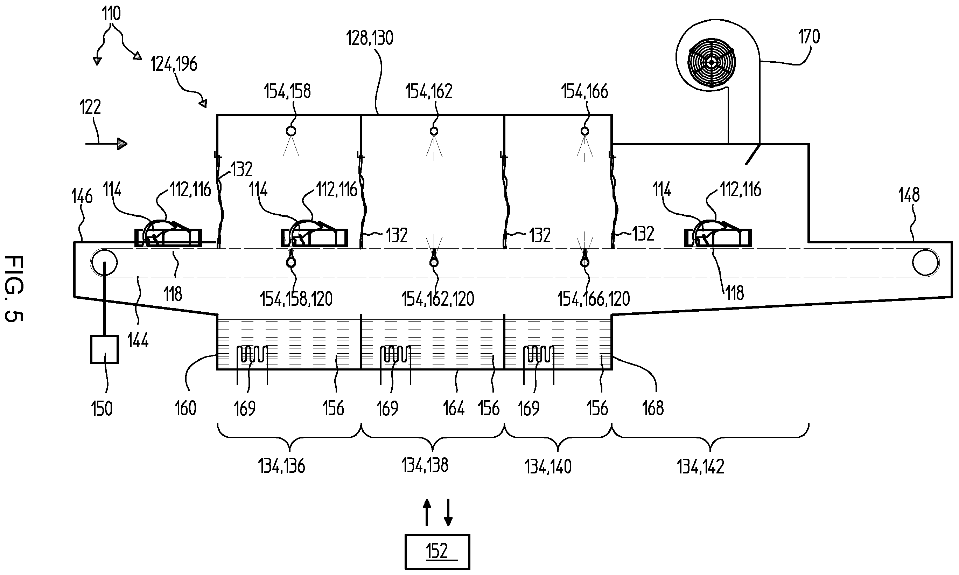

In dem Ausführungsbeispiel gemäß Figur 1 ist die Transportspülmaschine 124 exemplarisch als Bandspülmaschine 126 ausgestaltet. Der Reinigungsgut-Träger 118 kann dementsprechend beispielsweise als Vorrichtung ausgebildet sein, welche in ein Band oder eine Gliederkette der Transportvorrichtung 144 integrierbar ist, beispielsweise als Kettenglied, und/oder welche mit dem Band oder der Gliederkette der Transportvorrichtung 144 verbindbar ist, beispielsweise reversibel, beispielsweise durch eine formschlüssige und/oder kraftschlüssige Verbindung. So kann der Reinigungsgut-Träger 118, alternativ zu einer Integration in ein Band, auch beispielsweise in dieses Band eingehakt oder eingerastet werden. Alternativ kann die Transportspülmaschine 124 jedoch auch auf andere Weise ausgestaltet sein, beispielsweise als Korbtransport-Spülmaschine 196. Dies ist exemplarisch in den Figuren 5 und 6 gezeigt. Dabei zeigt Figur 5, analog zur Figur 1, ein Ausführungsbeispiel einer als Korbtransport-Spülmaschine 196 ausgestalteten Transportspülmaschine 124 in einer Schnittdarstellung mit Schnittebene parallel zu einer Transportrichtung 122. Figur 6 zeigt entsprechend eine Schnittdarstellung durch einen als Reinigungskorb 198 ausgestalteten Reinigungsgut-Träger 118 zum Einsatz in der Korbtransport-Spülmaschine 196 gemäß Figur 5. Beide Figuren werden im Folgenden gemeinsam beschrieben.

Das Reinigungssystem 110 gemäß Figur 5 weist wiederum, wie oben beschrieben, eine Transportspülmaschine 124 und eine Mehrzahl von Reinigungsgut-Trägern 118 auf. Die Transportspülmaschine 124 ist in dem dargestellten Ausführungsbeispiel als Korbtransport-Spülmaschine 196 ausgestaltet, und die Reinigungsgut-Träger 118 sind als Reinigungskörbe 198 ausgestaltet. Dementsprechend kann die Transportvorrichtung 144 insbesondere als Transportband für die Reinigungskörbe 198 ausgestaltet sein, auf welches die Reinigungskörbe 198 aufgesetzt werden können. Ansonsten kann bezüglich der möglichen Ausgestaltungen und der Elemente der Transportspülmaschine 124 weitgehend auf die Beschreibung der Figur 1 verwiesen werden.

In Figur 6 ist ein Ausführungsbeispiel eines Reinigungskorbs 198 in einer Schnittdarstellung gezeigt. Der Reinigungskorb 198 umfasst wiederum ein Basiselement 172, welches beispielsweise als Korbboden ausgestaltet sein kann. In dem Basiselement 172 können eine Mehrzahl von Öffnungen 200 vorgesehen sein, durch welche Reinigungsfluid aus dem Reinigungskorb 198 ablaufen kann. Weiterhin ist wiederum mindestens ein Düsenrohr 174 vorgesehen, welches beispielsweise wiederum auf einer Unterseite 182 des Reinigungsgut-Trägers 118 und/oder des Basiselements 172 eine Eintrittsöffnung 176 aufweisen kann. Wiederum sind außerdem ein Fangtrichter 180 sowie eine Austrittsdüse 178 vorgesehen, wobei auf die Beschreibung der Figuren 2 bis 4 verwiesen werden kann. Eine analoge Ausgestaltung zu diesen Figuren ist auch in Figur 6 grundsätzlich möglich. Weiterhin ist auch möglich, die Eintrittsöffnung 176 auf einer anderen Seite als der Unterseite 182 zu platzieren, wobei jedoch eine Platzierung auf der Unterseite 182 die oben beschriebenen Vorteile aufweisen kann.

Wie in Figur 6 erkennbar, umfasst das Reinigungsgut 112 Elemente persönlicher Schutzausrüstung. Exemplarisch ist hier wiederum ein Helm 116 dargestellt, welcher Atemschutzfunktion aufweisen kann. Dieser Helm 116 weist mindestens einen Hohlraum 114 auf, welcher in Figur 6 in Schnittdarstellung gezeigt ist. Dieser Hohlraum 114 ist als Luftführungskanal 202 ausgestaltet, auch als Atemluftkanal bezeichnet. Dieser Luftführungskanal 202, wobei ein oder mehrere derartige Kanäle vorgesehen sein können, kann zur Zufuhr von Einatemluft und/oder zur Abfuhr von Ausatemluft eingerichtet sein.

Auf dem Basiselement 172 kann wiederum mindestens ein Gestell 186 vorgesehen sein. Dieses Gestell 186 kann einerseits beispielsweise einen Rahmen 188 bilden oder umfassen, welcher beispielsweise eine Korbwand des Reinigungskorbs 198 bilden kann. Weiterhin kann das Gestell 186 beispielsweise mindestens eine Halterung 204 für das Reinigungsgut 112 bilden oder umfassen. Mittels dieser Halterung 204 kann beispielsweise das Reinigungsgut 112 derart fixiert werden, dass mindestens eine Einlassöffnung 206 des Hohlraums 114, beispielsweise des Luftführungskanals 202, mit der Austrittsdüse 178 zur Deckung kommt, wie in Figur 6 erkennbar. Auf diese Weise kann aus der Wurfdüse 120 austretendes Reinigungsfluid beispielsweise in die Einlassöffnung 206 des Hohlraums 114 eingespritzt werden. Der Hohlraum 114 kann darüber hinaus, beispielsweise an einer Stirnseite 208 des Helms 116, mindestens eine Auslassöffnung 210 aufweisen. Aus dieser Auslassöffnung 210 oder auch aus der Einlassöffnung 206 kann das eingespritzte Reinigungsfluid wieder aus dem Hohlraum 114 austreten. Somit kann der Hohlraum 114 allgemein mindestens eine Öffnung aufweisen.

Neben der Funktion einer Fixierung des Reinigungsguts 112 relativ zu der Austrittsdüse 178 kann auch eine anderweitige Fixierung des Reinigungsguts 112 erfolgen. So kann beispielsweise eine Bebänderung 212 des Helms 116 durch die Halterung 204 gehalten werden. Wiederum alternativ oder zusätzlich kann der Helm 116 auch beispielsweise mindestens ein Visier 214 aufweisen, welches für die Reinigung beispielsweise in einer bestimmten Stellung gehalten werden kann. So kann das Gestell 186 beispielsweise mindestens eine Halterung 216 für das Visier 214 aufweisen, welche das Visier 214 bei der Reinigung in einer vorgegebenen Stellung, beispielsweise einer vorgegebenen Winkelstellung, halten kann.

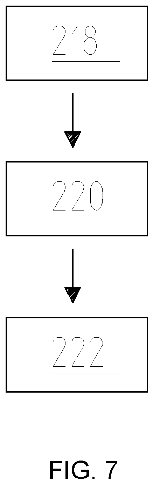

In Figur 7 ist ein schematischer Ablaufplan eines Ausführungsbeispiels eines Verfahrens zur Reinigung von Reinigungsgut 112 mit mindestens einem Hohlraum 114 gezeigt. Das Verfahren umfasst die Verwendung eines erfindungsgemäßen Reinigungssystems 110, beispielsweise gemäß Figur 1 oder gemäß Figur 5. Dementsprechend kann für mögliche Ausgestaltungen weitgehend auf die obige Beschreibung dieser Figuren verwiesen werden.

Das Verfahren umfasst in dem dargestellten Ausführungsbeispiel ein Aufnehmen des Reinigungsguts 112 in dem Reinigungsgut-Träger 118, derart, dass der Hohlraum 114 des Reinigungsguts 112 mittels der Austrittsdüse 178 des Düsenrohrs 174 mit dem mindestens einen Reinigungsfluid 156 beaufschlagbar ist (Schritt 218). Dies kann beispielsweise, wie oben ausgeführt, dadurch erfolgen, dass mindestens eine Einlassöffnung 206 des Hohlraums 114 mit der Austrittsdüse 178 zur Deckung gebracht wird.

Weiterhin umfasst das Verfahren gemäß Figur 7 einen Transport des Reinigungsgut-Trägers 118 mittels der Transportvorrichtung 144 durch die Reinigungskammer 128 (Schritt 220).

Weiterhin umfasst das Verfahren gemäß Figur 7 ein Beaufschlagen des Reinigungsguts 112 mit dem mindestens einen Reinigungsfluid 156 mittels der Beaufschlagungsvorrichtung 154 (Schritt 222). Diese Beaufschlagung erfolgt derart, dass in mindestens einer Stellung des Reinigungsgut-Trägers 118 relativ zu der mindestens einen Wurfdüse 120, aus der Wurfdüse 120 austretendes Reinigungsfluid 156 in den Fangtrichter 180 des Düsenrohrs 174 eintritt, insbesondere eingespritzt wird. Weiterhin erfolgt die Beaufschlagung derart, dass dieses Reinigungsfluid 156 durch das Düsenrohr 174 hindurchtritt und aus der Austrittsdüse 178 des Düsenrohrs 174 in den Hohlraum 114 des Reinigungsguts 112 eintritt, insbesondere eingespritzt wird. Diese Beaufschlagung kann insbesondere während einer Bewegung der Transportvorrichtung 144 erfolgen.

### Bezugszeichenliste

- 110: Reinigungssystem
- 112: Reinigungsgut
- 114: Hohlraum
- 116: Helm
- 118: Reinigungsgut-Träger
- 120: Wurfdüse
- 122: Transportrichtung
- 124: Transportspülmaschine
- 126: Bandspülmaschine
- 128: Reinigungskammer
- 130: Reinigungstunnel
- 132: Trennvorhang
- 134: Zone
- 136: Vorabräumzone
- 138: Spülzone
- 140: Klarspülzone
- 142: Trocknungszone
- 144: Transportvorrichtung
- 146: Einlaufbereich
- 148: Auslaufbereich
- 150: Antrieb
- 152: Steuerung
- 154: Beaufschlagungsvorrichtung
- 156: Reinigungsfluid
- 158: Vorabräumzonen-Düsensystem
- 160: Vorabräumtank
- 162: Spülzonen-Düsensystem
- 164: Spültank
- 166: Nachspüldüsensystem
- 168: Nachspültank
- 169: Heizvorrichtung
- 170: Gebläse
- 172: Basiselement
- 174: Düsenrohr
- 176: Eintrittsöffnung
- 178: Austrittsdüse
- 180: Fangtrichter
- 182: Unterseite
- 184: Oberseite
- 186: Gestell
- 188: Rahmen
- 190: Gitter
- 192: Gitterstrebe
- 194: Bandstange
- 196: Korbtransport-Spülmaschine
- 198: Reinigungskorb
- 200: Öffnung
- 202: Luftführungskanal
- 204: Halterung
- 206: Einlassöffnung
- 208: Stirnseite
- 210: Auslassöffnung
- 212: Bebänderung
- 214: Visier
- 216: Halterung für Visier
- 218: Aufnehmen des Reinigungsguts auf dem Reinigungsgut-Träger
- 220: Transport des Reinigungsgut-Trägers durch die Reinigungskammer
- 222: Beaufschlagen des Reinigungsguts mit mindestens einem Reinigungsfluid

## Patentansprüche

1. Verfahren zur Reinigung von Reinigungsgut (112) in Form persönlicher Schutzausrüstung ausgewählt aus der Gruppe bestehend aus einer Atemschutzmaske und einem Helm (116) mit mindestens einem Luftführungskanal (202), wobei das Reinigungsgut (112) mindestens einen Hohlraum (114) aufweist,
wobei mindestens ein Reinigungssystem (110) verwendet wird, umfassend:
I. mindestens eine Transportspülmaschine (124), umfassend:
- mindestens eine Reinigungskammer (128),
- mindestens eine Beaufschlagungsvorrichtung (154) zur Beaufschlagung des Reinigungsguts (112) in der Reinigungskammer (128) mit mindestens einem Reinigungsfluid (156),
- mindestens eine Transportvorrichtung (144) zum Transport des Reinigungsguts (112) von einem Einlauf (146) der Transportspülmaschine (124) durch die Reinigungskammer (128) hindurch zu einem Auslauf (148) der Transportspülmaschine (124); und
II. mindestens einen Reinigungsgut-Träger (118), umfassend mindestens ein Basiselement (172) sowie mindestens ein mit dem Basiselement (172) verbundenes Düsenrohr (174), wobei das Düsenrohr (174) mindestens eine Eintrittsöffnung (176) mit mindestens einem Fangtrichter (180) aufweist, wobei das Düsenrohr (174) mindestens eine Austrittsdüse (178) aufweist und wobei sich das Düsenrohr (174) von der Eintrittsöffnung (176) hin zu der Austrittsdüse (178) zumindest abschnittsweise verjüngt,
wobei das Reinigungsgut (112) derart auf dem Reinigungsgut-Träger (118) aufnehmbar ist, dass der Hohlraum (114) des Reinigungsguts (112) mit der Austrittsdüse (178) des Reinigungsgut-Trägers (118) in Verbindung steht, wobei der Reinigungsgut-Träger (118) mittels der Transportvorrichtung (144) durch die Reinigungskammer (128) transportierbar ist, wobei die Beaufschlagungsvorrichtung (154) mindestens eine stationäre Wurfdüse (120) aufweist, wobei die Wurfdüse (120) in mindestens einer Stellung des Reinigungsgut-Trägers (118) derart relativ zu dem Reinigungsgut-Träger (118) positioniert ist, dass aus der Wurfdüse (120) austretendes Reinigungsfluid (156) in den Fangtrichter (180) des Düsenrohrs (174) eintritt,
wobei das Verfahren folgende Schritte umfasst:
i) Aufnehmen des Reinigungsguts (112) auf dem Reinigungsgut-Träger (118), derart, dass der Hohlraum (114) des Reinigungsguts (112) mittels der Austrittsdüse (178) des Reinigungsgut-Trägers (118) mit mindestens einem Reinigungsfluid (156) beaufschlagbar ist;
ii) Transport des Reinigungsgut-Trägers (118) mittels der Transportvorrichtung (144) durch die Reinigungskammer (128); und
iii) Beaufschlagen des Reinigungsguts (112) mit mindestens einem Reinigungsfluid (156) mittels der Beaufschlagungsvorrichtung (154), wobei in mindestens einer Stellung des Reinigungsgut-Trägers (118) relativ zu der Wurfdüse (120) aus der Wurfdüse (120) austretendes Reinigungsfluid (156) in den Fangtrichter (180) des Düsenrohrs (174) eintritt, durch das Düsenrohr (174) hindurchtritt und aus der Austrittsdüse (178) des Düsenrohrs (174) in den Hohlraum (114) des Reinigungsguts (112) eintritt.

2. Verfahren nach dem vorhergehenden Anspruch, wobei das Reinigungsgut (112) mit einer Öffnung des Hohlraums (114) vor der Austrittsdüse (178) positioniert wird.

3. Verfahren nach einem der vorhergehenden Verfahrensansprüche, wobei auf dem Basiselement (172) mindestens ein Gestell (186) aufgebracht ist, wobei das Gestell (186) mindestens eine Halterung (204) für das Reinigungsgut (112) bildet, wobei mittels der Halterung (204) das Reinigungsgut (112) derart fixiert wird, dass mindestens eine Einlassöffnung (206) des Hohlraums (114) mit der Austrittsdüse (178) zur Deckung kommt, wobei aus der Wurfdüse (120) austretendes Reinigungsfluid (156) in die Einlassöffnung (206) des Hohlraums (114) eingespritzt wird, wobei der Hohlraum (114) weiterhin mindestens eine Auslassöffnung (210) aufweist, wobei das eingespritzte Reinigungsfluid (156) aus dieser Auslassöffnung (210) oder aus der Einlassöffnung (206) wieder aus dem Hohlraum (114) austritt.

4. Verfahren nach einem der vorhergehenden Verfahrensansprüche, wobei der Reinigungsgut-Träger (118) mehrteilig ausgebildet ist, wobei die Transportvorrichtung (144) als umlaufende Transportvorrichtung (144) ausgebildet ist, wobei der Reinigungsgut-Träger (118) mitsamt dem Reinigungsgut (112) mittels der Transportvorrichtung (144) durch die Reinigungskammer (128) transportiert wird, wobei anschließend das Reinigungsgut (112) sowie ein Teil des Reinigungsgut-Trägers (118) abgenommen werden und wobei ein weiterer Teil des Reinigungsgut-Trägers (118) mittels der Transportvorrichtung (144) wieder zurück zu dem Einlauf (146) transportiert werden.

5. Verfahren nach einem der vorhergehenden Verfahrensansprüche, wobei das Reinigungsgut (112) in dem Reinigungsgut-Träger (118) fixiert wird, derart, dass der mindestens eine Hohlraum (114) des Reinigungsguts (112) zu der Austrittsdüse (178) ortsfest positioniert ist.

6. Verfahren nach einem der vorhergehenden Verfahrensansprüche, wobei das Reinigungsgut (112) mindestens einen Helm (116) mit mindestens einem Visier (214) aufweist, wobei der Helm (116) in dem Reinigungsgut-Träger (118) fixiert wird, wobei weiterhin das Visier (214) in dem Reinigungsgut-Träger (118) in einer vorgegebenen Winkelstellung fixiert wird.

7. Verfahren nach einem der vorhergehenden Verfahrensansprüche, wobei die Transportspülmaschine (124) mehrere Wurfdüsen (120) aufweist, wobei mindestens zwei der Wurfdüsen (120) sequenziell mit dem Fangtrichter (180) in Wechselwirkung treten.

8. Verfahren nach einem der vorhergehenden Verfahrensansprüche, wobei die Transportspülmaschine (124) mindestens einen Sensor zur Erkennung des Reinigungsgut-Trägers (118) aufweist, wobei die Transportspülmaschine (124) weiterhin eine Steuerung (152) aufweist, wobei die Steuerung (152) eingerichtet ist, um eine Beaufschlagung der Wurfdüse (120) mit Reinigungsfluid (156) entsprechend der Erkennung des Reinigungsgut-Trägers (118) zu steuern.

9. Verfahren nach einem der vorhergehenden Verfahrensansprüche, wobei die Transportspülmaschine (124) mehrere Reinigungszonen (136, 138, 140) aufweist, wobei die Reinigungszonen (136, 138, 140) von dem Reinigungsgut (112) sequenziell durchlaufen werden, wobei die mindestens eine stationäre Wurfdüse (120) in mindestens einer der Reinigungszonen (136, 138, 140) angeordnet ist, wobei die Transportspülmaschine (124) weiterhin mindestens eine den Reinigungszonen (136, 138, 140) nachgelagerte Trocknungszone (142) aufweist, wobei in der Trocknungszone (142) mindestens eine Trocknungsdüse angeordnet ist, wobei die Trocknungsdüse in mindestens einer Stellung des Reinigungsgut-Trägers (118) innerhalb der Trocknungszone derart relativ zu dem Reinigungsgut-Träger (118) positioniert ist, dass aus der Trocknungsdüse austretende Trocknungsluft in den Fangtrichter (180) des Düsenrohrs (174) eintritt.

10. Verfahren nach einem der vorhergehenden Verfahrensansprüche, wobei die Transportvorrichtung (144) ausgewählt ist aus der Gruppe bestehend aus: einem Transportband, wobei der Reinigungsgut-Träger (118) auf das Transportband aufgesetzt wird; einem Transportband, wobei der Reinigungsgut-Träger (118) mit dem Transportband verbunden wird; als Gliederkette, wobei der Reinigungsgut-Träger (118) als Kettenglied in die Gliederkette eingefügt ist; einem Klinkentransportsystem, wobei der Reinigungsgut-Träger (118) auf Gleitelemente des Klinkentransportsystems aufsetzbar ist.

11. Verfahren nach einem der vorhergehenden Verfahrensansprüche, wobei die Wurfdüse (120) variabel ausgestaltet ist.

12. Reinigungsgut-Träger (118) zur Verwendung in einem Verfahren nach einem der vorhergehenden Ansprüche, umfassend mindestens ein Basiselement (172) sowie mindestens ein mit dem Basiselement (172) verbundenes Düsenrohr (174), wobei das Düsenrohr (174) mindestens eine Eintrittsöffnung (176) mit mindestens einem Fangtrichter (180) aufweist, wobei das Düsenrohr (174) mindestens eine Austrittsdüse (178) aufweist und wobei sich das Düsenrohr (174) von der Eintrittsöffnung (176) hin zu der Austrittsdüse (178) zumindest abschnittsweise verjüngt, wobei auf dem Basiselement (172) mindestens ein Gestell (186) aufgebracht ist, wobei das Gestell (186) mindestens eine Halterung (204) für das Reinigungsgut (112) bildet, wobei mittels der Halterung (204) das Reinigungsgut (112) derart fixierbar ist, dass mindestens eine Einlassöffnung (206) des Hohlraums (114) mit der Austrittsdüse (178) zur Deckung kommt.

13. Reinigungssystem (110) zur Durchführung des Verfahrens nach einem der vorhergehenden Verfahrensansprüche, umfassend:
I. mindestens eine Transportspülmaschine (124), umfassend:
- mindestens eine Reinigungskammer (128),
- mindestens eine Beaufschlagungsvorrichtung (154) zur Beaufschlagung des Reinigungsguts (112) in der Reinigungskammer (128) mit mindestens einem Reinigungsfluid (156),
- mindestens eine Transportvorrichtung (144) zum Transport des Reinigungsguts (112) von einem Einlauf (146) der Transportspülmaschine (124) durch die Reinigungskammer (128) hindurch zu einem Auslauf (148) der Transportspülmaschine (124); und
II. mindestens einen Reinigungsgut-Träger (118) nach dem vorhergehenden Anspruch,
wobei das Reinigungsgut (112) derart auf dem Reinigungsgut-Träger (118) aufnehmbar ist, dass der Hohlraum (114) des Reinigungsguts (112) mit der Austrittsdüse (178) des Reinigungsgut-Trägers (118) in Verbindung steht, wobei der Reinigungsgut-Träger (118) mittels der Transportvorrichtung (144) durch die Reinigungskammer (128) transportierbar ist, wobei die Beaufschlagungsvorrichtung (154) mindestens eine stationäre Wurfdüse (120) aufweist, wobei die Wurfdüse (120) in mindestens einer Stellung des Reinigungsgut-Trägers (118) derart relativ zu dem Reinigungsgut-Träger (118) positioniert ist, dass aus der Wurfdüse (120) austretendes Reinigungsfluid (156) in den Fangtrichter (180) des Düsenrohrs (174) eintritt.

## Claims

1. Method for cleaning items to be cleaned (112) in the form of personal protective equipment selected from the group composed of a respirator mask and a helmet (116) having at least one air-guiding duct (202), wherein the items to be cleaned (112) have at least one cavity (114),
wherein at least one cleaning system (110) comprising the following is used:
I. at least one conveyor washer (124), comprising:
- at least one cleaning chamber (128),
- at least one impingement device (154) for impinging the items to be cleaned (112) in the cleaning chamber (128) with at least one cleaning fluid (156);
- at least one transport device (144) for transporting the items to be cleaned (112) from an inlet (146) of the conveyor washer (124), through the cleaning chamber (128), to an outlet (148) of the conveyor washer (124); and
II. at least one cleaning items carrier (118) comprising at least one base element (172) as well as at least one nozzle tube (174) connected to the base element (172), wherein the nozzle tube (174) has at least one entry opening (176) having at least one funnel trap (180), wherein the nozzle tube (174) has at least one exit nozzle (178) and wherein the nozzle tube (174) at least in portions tapers from the entry opening (176) toward the exit nozzle (178),
wherein the items to be cleaned (112) are able to be received on the cleaning items carrier (118) in such a manner that the cavity (114) of the items to be cleaned (112) is connected to the exit nozzle (178) of the cleaning items carrier (118), wherein the cleaning items carrier (118) by means of the transport device (144) is transportable through the cleaning chamber (128), wherein the impingement device (154) has at least one stationary ejecting nozzle (120), wherein the ejecting nozzle (120) in at least one position of the cleaning items carrier (118) is positioned relative to the cleaning items carrier (118) in such a manner that cleaning fluid (156) exiting the ejecting nozzle (120) enters the funnel trap (180) of the nozzle tube (174);
wherein the method comprises the following steps:
i) receiving the items to be cleaned (112) on the cleaning items carrier (118) in such a manner that the cavity (114) of the items to be cleaned (112) by means of the exit nozzle (178) of the cleaning items carrier (118) is able to be impinged with at least one cleaning fluid (156);
ii) transporting the cleaning items carrier (118) through the cleaning chamber (128) by means of the transport device (144); and
iii)impinging the items to be cleaned (112) with at least one cleaning fluid (156) by means of the impingement device (154), wherein in at least one position of the cleaning items carrier (118) relative to the ejecting nozzle (120), cleaning fluid (156) exiting the ejecting nozzle (120) enters the funnel trap (180) of the nozzle tube (174), passes through the nozzle tube (174), and from the exit nozzle (178) of the nozzle tube (174) enters the cavity (114) of the items to be cleaned (112).

2. Method according to the preceding claim, wherein the items to be cleaned (112) by way of an opening of the cavity (114) are positioned in front of the exit nozzle (178).

3. Method according to one of the preceding method claims, wherein at least one rack (186) is attached to the base element (172), wherein the rack (186) forms at least one mounting (204) for the items to be cleaned (112), wherein the items to be cleaned (112) by means of the mounting (204) are fixed in such a manner that at least one inlet opening (206) of the cavity (114) comes to be congruent with the exit nozzle (178), wherein cleaning fluid (156) exiting the ejecting nozzle (120) is injected into the inlet opening (206) of the cavity (114), wherein the cavity (114) furthermore has at least one outlet opening (210), wherein the injected cleaning fluid (156) exits the cavity (114) again from this outlet opening (210) or from the inlet opening (206).

4. Method according to one of the preceding method claims, wherein the cleaning items carrier (118) is configured in multiple parts, wherein the transport device (144) is configured as a revolving transport device (144), wherein the cleaning items carrier (118) conjointly with the items to be cleaned (112) by means of the transport device (144) is transported through the cleaning chamber (128), wherein the items to be cleaned (112) as well as one part of the cleaning items carrier (118) are subsequently removed, and wherein a further part of the cleaning items carrier (118) by means of the transport device (144) is transported back to the inlet (146).

5. Method according to one of the preceding method claims, wherein the items to be cleaned (112) are fixed in the cleaning items carrier (118) in such a manner that the at least one cavity (114) of the items to be cleaned (112) is positioned so as to be locationally fixed in relation to the exit nozzle (178) .

6. Method according to one of the preceding method claims, wherein the items to be cleaned (112) have at least one helmet (116) having at least one visor (214), wherein the helmet (116) is fixed in the cleaning items carrier (118), wherein the visor (214) in the cleaning items carrier (118) is furthermore fixed at a predefined angular position.

7. Method according to one of the preceding method claims, wherein the conveyor washer (124) has a plurality of ejecting nozzles (120), wherein at least two of the ejecting nozzles (120) interact sequentially with the funnel trap (180).

8. Method according to one of the preceding method claims, wherein the conveyor washer (124) has at least one sensor for identifying the cleaning items carrier (118), wherein the conveyor washer (124) furthermore has a controller (152), wherein the controller (152) is specified to control an impingement of the ejecting nozzle (120) with cleaning fluid (156) in a manner corresponding to the identification of the cleaning items carrier (118).

9. Method according to one of the preceding method claims, wherein the conveyor washer (124) has a plurality of cleaning zones (136, 138, 140), wherein the cleaning zones (136, 138, 140) are passed sequentially by the items to be cleaned (112), wherein the at least one stationary ejecting nozzle (120) is disposed in at least one of the cleaning zones (136, 138, 140), wherein the conveyor washer (124) furthermore has at least one drying zone (142) which is disposed downstream of the cleaning zones (136, 138, 140), wherein at least one drying nozzle is disposed in the drying zone (142), wherein the drying nozzle in at least one position of the cleaning items carrier (118) within the drying zone is positioned relative to the cleaning items carrier (118) in such a manner that drying air exiting the drying nozzle enters the funnel trap (180) of the nozzle tube (174).

10. Method according to one of the preceding method claims, wherein the transport device (144) is selected from the group composed of: a transport belt, wherein the cleaning items carrier (118) is placed onto the transport belt; a transport belt, wherein the cleaning items carrier (118) is connected to the transport belt; as a link chain, wherein the cleaning items carrier (118) is inserted as a chain link in the link chain; a latching transport system, wherein the cleaning items carrier (118) is able to be placed on sliding elements of the latching transport system.

11. Method according to one of the preceding method claims, wherein the ejecting nozzle (120) is designed to be variable.

12. Cleaning items carrier (118) for use in a method according to one of the preceding claims, comprising at least one base element (172) as well as at least one nozzle tube (174) connected to the base element (172), wherein the nozzle tube (174) has at least one entry opening (176) having at least one funnel trap (180), wherein the nozzle tube (174) has at least one exit nozzle (178) and wherein the nozzle tube (174) at least in portions tapers from the entry opening (176) toward the exit nozzle (178), wherein at least one rack (186) is attached to the base element (172), wherein the rack (186) forms at least one mounting (204) for the items to be cleaned (112), wherein the items to be cleaned (112) by means of the mounting (204) are able to be fixed in such a manner that at least one inlet opening (206) of the cavity (114) comes to be congruent with the exit nozzle (178).

13. Cleaning system (110) for carrying out the method according to one of the preceding method claims, comprising:
I. at least one conveyor washer (124), comprising:
- at least one cleaning chamber (128);
- at least one impingement device (154) for impinging the items to be cleaned (112) in the cleaning chamber (128) with at least one cleaning fluid (156);
- at least one transport device (144) for transporting the items to be cleaned (112) from an inlet (146) of the conveyor washer (124), through the cleaning chamber (128), to an outlet (148) of the conveyor washer (124); and
II. at least one cleaning items carrier (118) according to the preceding claim,
wherein the items to be cleaned (112) are able to be received on the cleaning items carrier (118) in such a manner that the cavity (114) of the items to be cleaned (112) is connected to the exit nozzle (178) of the cleaning items carrier (118), wherein the cleaning items carrier (118) by means of the transport device (144) is transportable through the cleaning chamber (128), wherein the impingement device (154) has at least one stationary ejecting nozzle (120), wherein the ejecting nozzle (120) in at least one position of the cleaning items carrier (118) is positioned relative to the cleaning items carrier (118) in such a manner that cleaning fluid (156) exiting the ejecting nozzle (120) enters the funnel trap (180) of the nozzle tube (174).

## Revendications

1. Procédé de nettoyage d'un article à nettoyer (112) sous la forme d'un équipement de protection personnel sélectionné dans le groupe constitué d'un masque respiratoire protecteur et d'un casque (116) comportant au moins un canal de guidage d'air (202), l'article à nettoyer (112) présentant au moins une cavité (114),
au moins un système de nettoyage (110) étant utilisé, comprenant :
I. au moins un lave-vaisselle à convoyeur (124), comprenant :
- au moins une chambre de nettoyage (128),
- au moins un dispositif d'alimentation (154) servant à alimenter l'article à nettoyer (112) dans la chambre de nettoyage (128) en au moins un fluide de nettoyage (156),
- au moins un dispositif de transport (144) servant à transporter l'article à nettoyer (112) à partir d'une entrée (146) du lave-vaisselle à convoyeur (124) à travers la chambre de nettoyage (128) jusqu'à une sortie (148) du lave-vaisselle à convoyeur (124) ; et
II. au moins un support d'article à nettoyer (118), comprenant au moins un élément de base (172) ainsi qu'au moins un tube de buse (174) relié à l'élément de base (172), le tube de buse (174) présentant au moins une ouverture d'entrée (176) comportant au moins un entonnoir collecteur (180), le tube de buse (174) présentant au moins une buse de sortie (178) et le tube de buse (174) se rétrécissant au moins dans certaines parties à partir de l'ouverture d'entrée (176) en direction de la buse de sortie (178),
l'article à nettoyer (112) pouvant être reçu sur le support d'article à nettoyer (118) de telle sorte que la cavité (114) de l'article à nettoyer (112) soit en liaison avec la buse de sortie (178) du support d'article à nettoyer (118), le support d'article à nettoyer (118) pouvant être transporté à travers la chambre de nettoyage (128) au moyen du dispositif de transport (144), le dispositif d'alimentation (154) présentant au moins une buse d'éjection (120) fixe, la buse d'éjection (120) étant, dans au moins une position du support d'article à nettoyer (118), positionnée par rapport au support d'article à nettoyer (118) de telle sorte que le fluide de nettoyage (156) sortant de la buse d'éjection (120) entre dans l'entonnoir collecteur (180) du tube de buse (174),
le procédé comprenant les étapes suivantes :
i) réception de l'article à nettoyer (112) sur le support d'article à nettoyer (118), de telle sorte que la cavité (114) de l'article à nettoyer (112) puisse être alimentée en au moins un fluide de nettoyage (156) au moyen de la buse de sortie (178) du support d'article à nettoyer (118) ;
ii) transport du support d'article à nettoyer (118) au moyen du dispositif de transport (144) à travers la chambre de nettoyage (128) ; et
iii) alimentation de l'article à nettoyer (112) en au moins un fluide de nettoyage (156) au moyen du dispositif d'alimentation (154) et, dans au moins une position du support d'article à nettoyer (118) par rapport à la buse d'éjection (120), le fluide de nettoyage (156) sortant de la buse d'éjection (120) entrant dans l'entonnoir collecteur (180) du tube de buse (174), traversant le tube de buse (174) et entrant dans la cavité (114) de l'article à nettoyer (112) à partir de la buse de sortie (178) du tube de buse (174).

2. Procédé selon la revendication précédente, l'article à nettoyer (112) étant positionné de telle sorte qu'une ouverture de la cavité (114) soit devant la buse de sortie (178).

3. Procédé selon l'une des revendications précédentes, au moins un bâti (186) étant monté sur l'élément de base (172), le bâti (186) formant au moins un dispositif de retenue (204) pour l'article à nettoyer (112), l'article à nettoyer (112) étant fixé au moyen du dispositif de retenue (204), de telle sorte qu'au moins une ouverture d'entrée (206) de la cavité (114) vienne en coïncidence avec la buse de sortie (178), le fluide de nettoyage (156) sortant de la buse d'éjection (120) étant injecté dans l'ouverture d'entrée (206) de la cavité (114), la cavité (114) présentant en outre au moins une ouverture de sortie (210), le fluide de nettoyage (156) injecté sortant à nouveau de la cavité (114) à partir de cette ouverture de sortie (210) ou à partir de l'ouverture d'entrée (206).

4. Procédé selon l'une des revendications précédentes, le support d'article à nettoyer (118) étant réalisé en plusieurs parties, le dispositif de transport (144) étant réalisé sous forme de dispositif de transport (144) rotatif, le support d'article à nettoyer (118) étant transporté conjointement avec l'article à nettoyer (112) au moyen du dispositif de transport (144) à travers la chambre de nettoyage (128), ensuite l'article à nettoyer (112) ainsi qu'une partie du support d'article à nettoyer (118) étant retirés et une autre partie du support d'article à nettoyer (118) étant à nouveau transportée de manière à revenir à l'entrée (146) au moyen du dispositif de transport (144).

5. Procédé selon l'une des revendications précédentes, l'article à nettoyer (112) étant fixé dans le support d'article à nettoyer (118), de telle sorte que l'au moins une cavité (114) de l'article à nettoyer (112) soit positionnée de manière fixe par rapport à la buse de sortie (178).

6. Procédé selon l'une des revendications précédentes, l'article à nettoyer (112) présentant au moins un casque (116) comportant au moins une visière (214), le casque (116) étant fixé dans le support d'article à nettoyer (118), en outre la visière (214) étant fixée dans le support d'article à nettoyer (118) dans une position angulaire prédéfinie.

7. Procédé selon l'une des revendications précédentes, le lave-vaisselle à convoyeur (124) présentant plusieurs buses d'éjection (120), au moins deux des buses d'éjection (120) interagissant séquentiellement avec l'entonnoir collecteur (180).

8. Procédé selon l'une des revendications précédentes, le lave-vaisselle à convoyeur (124) présentant au moins un capteur servant à détecter le support d'article à nettoyer (118), le lave-vaisselle à convoyeur (124) présentant en outre une commande (152), la commande (152) étant conçue pour commander une alimentation de la buse d'éjection (120) en fluide de nettoyage (156) de manière correspondante à la détection du support d'article à nettoyer (118).

9. Procédé selon l'une des revendications précédentes, le lave-vaisselle à convoyeur (124) présentant plusieurs zones de nettoyage (136, 138, 140), les zones de nettoyage (136, 138, 140) étant traversées séquentiellement par l'article à nettoyer (112), l'au moins une buse d'éjection fixe (120) étant disposée dans au moins l'une des zones de nettoyage (136, 138, 140), le lave-vaisselle à convoyeur (124) présentant en outre au moins une zone de séchage (142) placée en aval des zones de nettoyage (136, 138, 140), au moins une buse de séchage étant disposée dans la zone de séchage (142), la buse de séchage, dans au moins une position du support d'article à nettoyer (118) à l'intérieur de la zone de séchage, étant positionnée par rapport au support d'article à nettoyer (118) de telle sorte que l'air de séchage sortant de la buse de séchage entre dans l'entonnoir collecteur (180) du tube de buse (174).

10. Procédé selon l'une des revendications précédentes, le dispositif de transport (144) étant sélectionné dans le groupe constitué de : une bande transporteuse, le support d'article à nettoyer (118) étant placé sur la bande transporteuse ; une bande transporteuse, le support d'article à nettoyer (118) étant relié à la bande transporteuse ; sous forme de chaîne à maillons, le support d'article à nettoyer (118) étant inséré en tant que maillon de chaîne dans la chaîne à maillons ; un système de transport à cliquets, le support d'article à nettoyer (118) pouvant être placé sur des éléments de glissement du système de transport à cliquets.

11. Procédé selon l'une des revendications précédentes, la buse d'éjection (120) étant configurée de manière variable.

12. Support d'article à nettoyer (118) destiné à être utilisé dans un procédé selon l'une des revendications précédentes, comprenant au moins un élément de base (172) ainsi qu'au moins un tube de buse (174) relié à l'élément de base (172), le tube de buse (174) présentant au moins une ouverture d'entrée (176) comportant au moins un entonnoir collecteur (180), le tube de buse (174) présentant au moins une buse de sortie (178) et le tube de buse (174) se rétrécissant au moins dans certaines parties à partir de l'ouverture d'entrée (176) en direction de la buse de sortie (178), au moins un bâti (186) étant monté sur l'élément de base (172), le bâti (186) formant au moins un dispositif de retenue (204) pour l'article à nettoyer (112), l'article à nettoyer (112) pouvant être fixé au moyen du dispositif de retenue (204), de telle sorte qu'au moins une ouverture d'entrée (206) de la cavité (114) vienne en coïncidence avec la buse de sortie (178).

13. Système de nettoyage (110) servant à la mise en œuvre du procédé selon l'une des revendications précédentes, comprenant :
I. au moins un lave-vaisselle à convoyeur (124), comprenant :
- au moins une chambre de nettoyage (128),
- au moins un dispositif d'alimentation (154) servant à alimenter l'article à nettoyer (112) dans la chambre de nettoyage (128) en au moins un fluide de nettoyage (156),
- au moins un dispositif de transport (144) servant à transporter l'article à nettoyer (112) à partir d'une entrée (146) du lave-vaisselle à convoyeur (124) à travers la chambre de nettoyage (128) jusqu'à une sortie (148) du lave-vaisselle à convoyeur (124) ; et
II. au moins un support d'article à nettoyer (118) selon la revendication précédente,
l'article à nettoyer (112) pouvant être reçu sur le support d'article à nettoyer (118) de telle sorte que la cavité (114) de l'article à nettoyer (112) soit en liaison avec la buse de sortie (178) du support d'article à nettoyer (118), le support d'article à nettoyer (118) pouvant être transporté à travers la chambre de nettoyage (128) au moyen du dispositif de transport (144), le dispositif d'alimentation (154) présentant au moins une buse d'éjection (120) fixe, la buse d'éjection (120) étant, dans au moins une position du support d'article à nettoyer (118), positionnée par rapport au support d'article à nettoyer (118) de telle sorte que le fluide de nettoyage (156) sortant de la buse d'éjection (120) entre dans l'entonnoir collecteur (180) du tube de buse (174) .
